(19) Europäisches Patentamt
European Patent Office
Office européen des brevets

(11) Veröffentlichungsnummer : **0 435 827 A2**

# (12) EUROPÄISCHE PATENTANMELDUNG

(21) Anmeldenummer : **90811005.9**

(22) Anmeldetag : **19.12.90**

(51) Int. Cl.[5] : **C07D 403/12**, C07D 403/10, C07D 239/36, A61K 31/505

(30) Priorität : **28.12.89 CH 4663/89**

(43) Veröffentlichungstag der Anmeldung : **03.07.91 Patentblatt 91/27**

(84) Benannte Vertragsstaaten : **AT BE CH DE DK ES FR GB GR IT LI LU NL SE**

(71) Anmelder : **CIBA-GEIGY AG**
**Klybeckstrasse 141**
**CH-4002 Basel (CH)**

(72) Erfinder : **Herold, Peter, Dr.**
**Mattweg 19**
**CH-4144 Arlesheim (CH)**
Erfinder : **Bühlmayer, Peter, Dr.**
**Hangstrasse 18**
**CH-4144 Arlesheim (CH)**

## (54) Diazaverbindungen.

(57) Diazaverbindungen der Formel

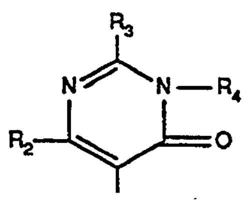

(I),

worin einer der Reste $R_1$ und $R_2$ einen gegebenenfalls durch Halogen oder Hydroxy substituierten aliphatischen Kohlenwasserstoffrest oder einen cycloaliphatischen oder araliphatischen Kohlenwasserstoffrest bedeutet, der andere der Reste $R_1$ und $R_2$ für die Gruppe der Formel

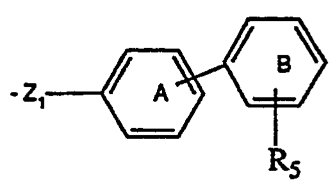

(Ia)

steht, in der $Z_1$ für Alkylen, O, $S(O)_m$ oder N(R) steht, $R_5$ Carboxy, Halogenalkansulfonylamino, $SO_3H$, $PO_2H_2$, $PO_3H_2$ oder 5-Tetrazolyl bedeutet und die Ringe A und B unabhängig voneinander gegebenenfalls substituiert sind durch einen aliphatischen Kohlenwasserstoffrest, der gegebenenfalls durch O unterbrochen und gegebenenfalls durch Hydroxy oder Halogen substituiert ist, gegebenenfalls durch einen aliphatischen Alkohol verethertes Hydroxy, Halogen, gegebenenfalls verestertes oder amidiertes Carboxy oder 5-Tetrazolyl, und entweder $R_3$ Halogen, Acyl, einen aromarischen Kohlenwasserstoffrest, gegebenenfalls verestertes oder amidiertes Carboxy, Cyano, $SO_3H$, $PO_3H_2$, $PO_2H_2$, 5-Tetrazolyl, gegebenenfalls substituiertes Sulfamoyl oder Acylamino bedeutet oder für $-Z_2-R'$ steht, worin $Z_2$ für eine Bindung oder für O, $S(O)_m$ oder N(R) steht und R' Wasserstoff oder einen aliphatischen Kohlenwasserstoffrest bedeutet, der gegebenenfalls durch O oder $S(O)_m$ unterbrochen ist und gegebenenfalls durch Halogen, Hydroxy, gegebenenfalls substituiertes Amino oder gegebenenfalls verestertes oder amidiertes Carboxy substituiert ist, und $R_4$ einen gegebenenfalls durch O oder $S(O)_m$ unterbrochenen aliphatischen Kohlenwasserstoffrest bedeutet, der gegebenenfalls durch gegebenenfalls verestertes oder amidiertes Carboxy, gegebenenfalls mit einem aromatischen Alkohol verethertes Hydroxy, gegebenenfalls substituiertes Amino, $S(O)_m$-R oder einen aromatischen Kohlenwasserstoffrest substituiert ist, oder $R_3$ und $R_4$ gemeinsam für Alkylen stehen, wobei R jeweils Wasserstoff oder einen aliphatischen Kohlenwasserstoffrest bedeutet und m jeweils für 0, 1 oder 2 steht, in freier Form oder in Salzform, können als Arzneimittelwirkstoffe verwendet werden und sind in an sich bekannter Weise herstellbar.

EP 0 435 827 A2

# DIAZAVERBINDUNGEN

Die Erfindung betrifft Diazaverbindungen der Formel

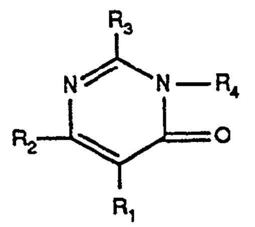

(I),

worin einer der Reste $R_1$ und $R_2$ einen gegebenenfalls durch Halogen oder Hydroxy substituierten aliphatischen Kohlenwasserstoffrest oder einen cycloaliphatischen oder araliphatischen Kohlenwasserstoffrest bedeutet, der andere der Reste $R_1$ und $R_2$ für die Gruppe der Formel

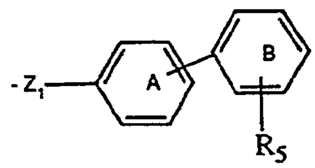

(Ia)

steht, in der $Z_1$ für Alkylen, O, $S(O)_m$ oder N(R) steht, $R_5$ Carboxy, Halogenalkansulfonylamino, $SO_3H$, $PO_2H_2$, $PO_3H_2$ oder 5-Tetrazolyl bedeutet und die Ringe A und B unabhängig voneinander gegebenenfalls substituiert sind durch einen aliphatischen Kohlenwasserstoffrest, der gegebenenfalls durch O unterbrochen und gegebenenfalls durch Hydroxy oder Halogen substituiert ist, gegebenenfalls durch einen aliphatischen Alkohol verethertes Hydroxy, Halogen, gegebenenfalls verestertes oder amidiertes Carboxy oder 5-Tetrazolyl, und entweder $R_3$ Halogen, Acyl, einen aromatischen Kohlenwasserstoffrest, gegebenenfalls verestertes oder amidiertes Carboxy, Cyano, $SO_3H$, $PO_3H_2$, $PO_2H_2$, 5-Tetrazolyl, gegebenenfalls substituiertes Sulfamoyl oder Acylamino bedeutet oder für $-Z_2-R'$ steht, worin $Z_2$ für eine Bindung oder für O, $S(O)_m$ oder N(R) steht und R' Wasserstoff oder einen aliphatischen Kohlenwasserstoffrest bedeutet, der gegebenenfalls durch O oder $S(O)_m$ unterbrochen ist und gegebenenfalls durch Halogen, Hydroxy, gegebenenfalls substituiertes Amino oder gegebenenfalls verestertes oder amidiertes Carboxy substituiert ist, und $R_4$ einen gegebenenfalls durch O oder $S(O)_m$ unterbrochenen aliphatischen Kohlenwasserstoffrest bedeutet, der gegebenenfalls durch gegebenenfalls verestertes oder amidiertes Carboxy, gegebenenfalls mit einem aromatischen Alkohol verethertes Hydroxy, gegebenenfalls substituiertes Amino, $S(O)_m$-R oder einen aromatischen Kohlenwasserstoffrest substituiert ist, oder $R_3$ und $R_4$ gemeinsam für Alkylen stehen, wobei R jeweils Wasserstoff oder einen aliphatischen Kohlenwasserstoffrest bedeutet und m jeweils für 0, 1 oder 2 steht, in freier Form oder in Salzform, ein Verfahren zur Herstellung dieser Verbindungen, die Verwendung dieser Verbindungen und pharmazeutische Präparate, enthaltend eine solche Verbindung I in freier Form oder in Form eines pharmazeutisch verwendbaren Salzes.

Die Verbindungen I können als, insbesondere pharmazeutisch verwendbare, Salze vorliegen. Weisen die Verbindungen I z. B. mindestens ein basisches Zentrum auf, können sie Säureadditionssalze bilden. Diese werden beispielsweise mit starken anorganischen Säuren, wie Mineralsäuren, z.B. Schwefelsäure, einer Phosphorsäure oder einer Halogenwasserstoffsäure, mit starken organischen Carbonsäuren, wie gegebenenfalls, z.B. durch Halogen, substituierten $C_1$-$C_4$-Alkancarbonsäuren, z.B. Essigsäure, wie gegebenenfalls ungesättigten Dicarbonsäuren, z.B. Oxal-, Malon-, Bernstein-, Malein-, Fumar-, Phthal- oder Terephthalsäure, wie Hydroxycarbonsäuren, z.B. Ascorbin-, Glykol-, Milch-, Äpfel-, Wein- oder Zitronensäure, wie Aminosäuren, z.B. Asparagin- oder Glutaminsäure, oder wie Benzoesäure, oder mit organischen Sulfonsäuren, wie gegebenenfalls, z.B. durch Halogen, substituierten $C_1$-$C_4$-Alkan- oder Aryl-sulfonsäuren, z.B. Methan- oder p-Toluolsulfonsäure, gebildet. Entsprechende Säureadditionssalze können auch mit einem gegebenenfalls zusätzlich vorhandenen basischen Zentrum gebildet werden. Ferner können die Verbindungen I mit mindestens einer aciden Gruppe (beispielsweise COOH oder 5-Tetrazolyl) Salze mit Basen bilden. Geeignete Salze mit Basen sind beispielsweise Metallsalze, wie Alkali- oder Erdalkalimetallsalze, z.B. Natrium-, Kalium- oder Magnesiumsalze, oder Salze mit Ammoniak oder einem organischen Amin, wie Morpholin, Thiomorpholin, Piperidin, Pyrrolidin, einem Mono-, Di- oder Triniederalkylamin, z. B. Ethyl-, tert.-Butyl-, Diethyl-, Diisopropyl-, Triethyl-, Tributyl- oder

Dimethyl-propyl-amin, oder einem Mono-, Di- oder Trihydroxyniederalkylamin, z.B. Mono-, Di- oder Triethanolamin. Weiterhin können entsprechende innere Salze gebildet werden. Umfasst sind ferner für pharmazeutische Verwendungen nicht geeignete Salze, die beispielsweise für die Isolierung bzw. Reinigung von freien Verbindungen I oder deren pharmazeutisch verwendbaren Salzen eingesetzt werden.

Ein aromatischer Kohlenwasserstoffrest ist insbesondere Phenyl.

Acyl bedeutet insbesondere Niederalkanoyl.

Verestertes Carboxy bedeutet beispielsweise Carboxy, welches durch einen aliphatischen Alkohol verestert ist, der sich von einem aliphatischen Kohlenwasserstoffrest ableitet, wie von Niederalkyl, Niederalkenyl oder in zweiter Linie Niederalkinyl, welcher gegebenenfalls durch O unterbrochen ist, wie von Niederalkoxyniederalkyl, -niederalkenyl oder -niederalkinyl.

Amidiertes Carboxy ist beispielsweise Carbamoyl, in welchem die Aminogruppe gegebenenfalls durch einen aliphatischen oder araliphatischen Kohlenwasserstoffrest, wie Niederalkyl, Niederalkenyl, Niederalkinyl oder Phenyl-niederalkyl, -niederalkenyl oder -niederalkinyl, unabhängig voneinander mono- oder disubstituiert oder durch einen zweiwertigen aliphatischen Kohlenwasserstoffrest, der gegebenenfalls durch O unterbrochen ist, wie Niederalkylen oder Niederalkylenoxyniederalkylen, disubstituiert ist.

Substituiertes Amino ist beispielsweise durch einen aliphatischen oder araliphatischen Kohlenwasserstoffrest, wie Niederalkyl, Niederalkenyl, Niederalkinyl oder Phenyl-niederalkyl, -niederalkenyl oder -niederalkinyl, unabhängig voneinander mono- oder disubstituiertes oder durch einen zweiwertigen aliphatischen Kohlenwasserstoffrest, der gegebenenfalls durch O unterbrochen ist, wie Niederalkylen oder Niederalkylenoxyniederalkylen, disubstituiertes Amino. Als Beispiele seien Niederalkyl-, Niederalkenyl-, Niederalkinyl-, Phenylniederalkyl-, Phenylniederalkenyl-, Phenylniederalkinyl-, Diniederalkyl-, N-Niederalkyl-N-phenylniederalkyl- und Di(phenylniederalkyl)amino genannt.

Ein aliphatischer Kohlenwasserstoffrest ist beispielsweise Niederalkyl, Niederalkenyl oder in zweiter Linie Niederalkinyl.

Ein aliphatischer Kohlenwasserstoffrest, der durch O unterbrochen ist, ist insbesondere Niederalkoxy-niederalkyl, -niederalkenyl oder -niederalkinyl, Niederalkenyloxy-niederalkyl, -niederalkenyl oder -niederalkinyl oder Niederalkoxyniederalkoxy- niederalkyl, während ein aliphatischer Kohlenwasserstoffrest, der durch $S(O)_m$ unterbrochen ist, insbesondere Niederalkylthio-niederalkyl, -niederalkenyl oder -niederalkinyl, Niederalkansulfinylniederalkyl oder -sulfonylniederalkyl, Niederalkenyl-thioniederalkyl, -sulfinylniederalkyl oder -sulfonylniederalkyl oder Niederalkinyl-thioniederalkyl, -sulfinylniederalkyl oder -sulfonylniederalkyl ist.

Ein durch Halogen oder Hydroxy substituierter aliphatischer Kohlenwasserstoffrest ist beispielsweise Halogen-niederalkyl, -niederalkenyl oder -niederalkinyl oder Hydroxy-niederalkyl, -niederalkenyl oder -niederalkinyl.

Ein durch Halogen oder Hydroxy substituierter aliphatischer Kohlenwasserstoffrest, der durch O oder $S(O)_m$ unterbrochen ist, ist ein entsprechender vorstehend angegebener Rest, der durch Halogen oder Hydroxy substituiert ist.

Ein durch gegebenenfalls substituiertes Amino, $S(O)_m$-R, einen aromatischen Kohlenwasserstoffrest, mit einem aromatischen Alkohol verethertes Hydroxy oder gegebenenfalls verestertes oder amidiertes Carboxy substituierter aliphatischer Kohlenwasserstoffrest, der gegebenenfalls durch O oder $S(O)_m$ unterbrochen ist, ist ein entsprechender vorstehend angegebener Rest, der durch Amino, vorstehend angegebenes substituiertes Amino, $S(O)_m$-R, einen vorstehend angegebenen aromatischen Kohlenwasserstoffrest, nachstehend angegebenes mit einem aromatischen Alkohol verethertes Hydroxy, Carboxy, vorstehend angegebenes verestertes Carboxy oder vorstehend angegebenes amidiertes Carboxy substituiert ist.

Ein cycloaliphatischer Kohlenwasserstoffrest ist beispielsweise Cycloalkyl oder in zweiter Linie Cycloalkenyl.

Als araliphatische Kohlenwasserstoffreste kommen insbesondere Phenylniederalkyl, ferner Phenyl-niederalkenyl und -niederalkinyl in Frage.

Mit einem aliphatischen Alkohol verethertes Hydroxy ist insbesondere Niederalkoxy oder Niederalkenyloxy.

Alkylen ist Methylen oder Niederalkylen.

Substituiertes Sulfamoyl bedeutet Niederalkyl- oder Diniederalkyl-sulfamoyl.

In Acylamino leitet sich Acyl von einer organischen Carbonsäure oder einer organischen Sulfonsäure ab. Beispielhaft seien als entsprechendes Acyl Niederalkanoyl, gegebenenfalls substituiertes Benzoyl, Niederalkansulfonyl, Halogenniederalkansulfonyl oder gegebenenfalls substituiertes Benzolsulfonyl genannt.

Mit einem aromatischen Alkohol verethertes Hydroxy ist insbesondere Phenoxy, ferner Naphthyloxy.

Vor- und nachstehend sind ungesättigte aliphatische, cycloaliphatische und araliphatische Substituenten in erster Linie nicht über ein C-Atom, von dem eine Mehrfachbindung ausgeht, mit einem aromatischen Rest verknüpft.

Aromatische Reste sind, sofern nicht abweichend definiert, jeweils unsubstituiert oder ein- oder mehrfach, z.B. zwei- oder dreifach, z.B. durch (einen) Substituenten ausgewählt aus der Gruppe bestehend aus Niederalkyl, Niederalkoxy, Halogen, Trifluormethyl und Hydroxy, substituiert.

Die Ringe A und B bilden einen Biphenylylrest, wobei entsprechendes 4-Biphenylyl bevorzugt ist.

Die vor- und nachstehend verwendeten Allgemeinbegriffe haben, sofern nicht abweichend definiert, folgende Bedeutungen :

Der Ausdruck "Nieder" bedeutet, dass entsprechende Gruppen und Verbindungen jeweils insbesondere bis und mit 7, vorzugsweise bis und mit 4, Kohlenstoffatome enthalten.

Niederalkansulfonyl bedeutet insbesondere $C_1$-$C_7$-Alkansulfonyl und ist z.B. Methan-, Ethan-, n-Propan- oder Isopropansulfonyl. Bevorzugt ist $C_1$-$C_4$-Alkansulfonyl. Halogenniederalkansulfonyl ist Halogen-$C_1$-$C_7$-alkansulfonyl, wie Trifluormethansulfonyl.

Niederalkansulfamoyl ist $C_1$-$C_7$-Alkansulfamoyl, wie Methan-, Ethan-, n-Propan-, Isopropan-, n-Butan-, sek.-Butan- oder tert.-Butansulfamoyl. Bevorzugt ist $C_1$-$C_4$-Alkansulfamoyl. Diniederalkansulfamoyl bedeutet Di-$C_1$-$C_7$-alkansulfamoyl, wie Dimethan-, Methan-ethan- oder Di-(n-propan)-sulfamoyl. Bevorzugt ist Di-$C_1$-$C_4$-alkansulfamoyl.

Halogen ist insbesondere Halogen mit einer Atomnummer bis und mit 35, d. h. Fluor, Chlor oder Brom, und umfasst ferner Iod.

Halogenalkansulfonylamino bedeutet insbesondere Halogen-$C_1$-$C_7$-alkansulfonylamino und ist z.B. Difluormethan-, Trifluormethan-, 2-Chlorethan-, 1,1,2-Trifluorethan-, 1,1,2-Trichlorethan-, Pentafluorethan- oder Heptafluorpropan-sulfonylamino. Bevorzugt ist Halogen-$C_1$ -$C_4$-alkansulfonylamino.

Niederalkanoyl bedeutet insbesondere $C_1$-$C_7$-Alkanoyl und ist z.B. Formyl, Acetyl, Propionyl, Butyryl, Isobutyryl oder Pivaloyl. Bevorzugt ist $C_2$-$C_5$-Alkanoyl.

Niederalkyl ist insbesondere $C_1$-$C_7$-Alkyl, d. h. Methyl, Ethyl, n-Propyl, Isopropyl, n-Butyl, Isobutyl, sek.-Butyl, tert.-Butyl oder ein entsprechender Pentyl-, Hexyl- oder Heptylrest. Bevorzugt ist $C_1$-$C_4$-Alkyl.

Niederalkenyl bedeutet insbesondere $C_3$-$C_7$-Alkenyl und ist z.B. Propen-2-yl, Allyl oder But-1-en-3-yl, -1-en-4-yl, -2-en-1-yl oder -2-en-2-yl. Bevorzugt ist $C_3$-$C_5$-Alkenyl.

Niederalkinyl ist insbesondere $C_3$-$C_7$-Alkinyl und bedeutet vorzugsweise Propargyl.

Niederalkoxy ist insbesondere $C_1$-$C_7$-Alkoxy d. h. Methoxy, Ethoxy, n-Propyloxy, Isopropyloxy, n-Butyloxy, Isobutyloxy, sek.-Butyloxy, tert.-Butyloxy oder entsprechendes Pentyloxy, Hexyloxy oder Heptyloxy. Bevorzugt ist $C_1$-$C_4$-Alkoxy.

Niederalkoxyniederalkyl bedeutet insbesondere $C_1$-$C_4$-Alkoxy-$C_1$-$C_4$-alkyl, wie 2-Methoxyethyl, 2-Ethoxyethyl, 2-(n-Propyloxy)ethyl oder Ethoxymethyl.

Niederalkoxy-niederalkenyl bzw. -niederalkinyl bedeutet insbesondere $C_1$-$C_4$-Alkoxy-$C_3$-$C_5$-alkenyl bzw. -alkinyl.

Niederalkenyloxy bedeutet insbesondere $C_3$-$C_7$-Alkenyloxy und ist z.B. Allyloxy, But-2-en-1-yloxy oder But-3-en-1-yloxy. Bevorzugt ist $C_3$-$C_5$-Alkenyloxy.

Halogenniederalkyl bedeutet insbesondere Halogen-$C_1$-$C_4$-alkyl, wie Trifluormethyl, 1,1,2-Trifluor-2-chlorethyl, Chlormethyl oder n-Heptafluorpropyl.

Halogenniederalkenyl bedeutet insbesondere Halogen-$C_3$-$C_5$-alkenyl, wie 3-Chlorallyl.

Halogenniederalkinyl ist insbesondere Halogen-$C_3$-$C_5$-alkinyl, wie 3-Chlorpropargyl.

Hydroxyniederalkyl bedeutet insbesondere Hydroxy-$C_1$-$C_4$-alkyl, wie Hydroxymethyl, 2-Hydroxyethyl oder 3-Hydroxypropyl.

Hydroxyniederalkenyl bedeutet insbesondere Hydroxy-$C_3$-$C_5$-alkenyl, wie 3-Hydroxyallyl.

Hydroxyniederalkinyl bedeutet insbesondere Hydroxy-$C_3$-$C_5$-alkinyl, wie 3-Hydroxypropargyl.

Phenylniederalkyl ist insbesondere Phenyl-$C_1$-$C_4$-alkyl und bedeutet vorzugsweise Benzyl oder 1- oder 2-Phenethyl, während Phenylniederalkenyl bzw. Phenylniederalkinyl insbesondere Phenyl-$C_3$-$C_5$-alkenyl bzw. -alkinyl bedeuten, insbesondere 3-Phenylallyl oder 3-Phenylpropargyl.

Niederalkylen bedeutet insbesondere $C_2$-$C_7$-Alkylen, ist geradkettig oder verzweigt und bedeutet insbesondere Ethylen, 1,3-Propylen, 1,4-Butylen, 1,2-Propylen, 2-Methyl-1,3-propylen oder 2,2-Dimethyl-1,3-propylen. Bevorzugt ist $C_2$-$C_5$-Alkylen.

Niederalkylenoxyniederalkylen bedeutet insbesondere $C_2$-$C_4$-Alkylenoxy-$C_2$-$C_4$-alkylen, vorzugsweise Ethylenoxyethylen.

Niederalkylamino bedeutet insbesondere $C_1$-$C_7$-Alkylamino und ist z.B. Methyl-, Ethyl-, n-Propyl- oder Isopropyl-amino. Bevorzugt ist $C_1$-$C_4$-Alkylamino.

Niederalkenylamino bedeutet vorzugsweise $C_3$-$C_5$-Alkenylamino, wie Allyl- oder Methallyl-amino.

Niederalkinylamino bedeutet vorzugsweise $C_3$-$C_5$-Alkinylamino, wie Propargylamino

Phenylniederalkylamino bedeutet vorzugsweise Phenyl-$C_1$-$C_4$-alkylamino, insbesondere Benzyl- oder 1- oder 2-Phenylethyl-amino.

Phenylniederalkenylamino bedeutet vorzugsweise Phenyl-$C_3$-$C_5$-alkenylamino, insbesondere Phenylallylamino oder 3-Phenylmethallylamino.

Phenylniederalkinylamino bedeutet vorzugsweise Phenyl-$C_3$-$C_5$-alkinylamino, insbesondere Phenylpropargylamino.

Diniederalkylamino bedeutet insbesondere Di-$C_1C_4$-alkylamino, wie Dimethyl-, Diethyl-, Di(n-propyl)-, Methyl-propyl-, Methyl-ethyl-, Methyl-butyl- oder Dibutyl-amino.

N-Niederalkyl-N-phenylniederalkyl-amino bedeutet insbesondere N-$C_1$-$C_4$-Alkyl-N-phenyl-$C_1$-$C_4$-alkylamino, vorzugsweise Methyl-benzyl-amino oder Ethyl-benzyl-amino.

Di(phenylniederalkyl)amino bedeutet insbesondere Di(phenyl-$C_1$-$C_4$-alkyl)amino, vorzugweise Dibenzylamino.

Niederalkenyloxyniederalkyl bedeutet insbesondere $C_3$-$C_5$-Alkenyloxy-$C_1$-$C_4$-alkyl, wie 2-Allyloxyethyl, und Niederalkenyloxy-niederalkenyl bzw. -niederalkinyl bedeutet insbesondere $C_3$-$C_5$-Alkenyloxy-$C_3$-$C_5$-alkenyl bzw. -alkinyl.

Niederalkylthio-niederalkenyl bzw. -niederalkinyl bedeutet insbesondere $C_1$-$C_4$-Alkylthio-$C_3$-$C_5$-alkenyl bzw. -alkinyl.

Niederalkylthioniederalkyl bedeutet insbesondere $C_1$-$C_4$-Alkylthio-$C_1$-$C_4$-alkyl, wie Ethylthiomethyl, 2-Ethylthioethyl, 2-Methylthioethyl oder 2-Isopropylthioethyl, während als Niederalkan-sulfinylniederalkyl bzw. -sulfonylniederalkyl insbesondere entsprechende $C_1$-$C_4$-Alkan-sulfinyl-$C_1$-$C_4$-alkylreste bzw. -sulfonyl-$C_1$-$C_4$-alkylreste in Frage kommen.

Niederalkenylthioniederalkyl ist insbesondere $C_3$-$C_5$-Alkenylthio-$C_1$-$C_4$-alkyl, wie 1-Allylthioethyl or 3-Allylthiopropyl, waehrend Niederalkenyl-sulfinylniederalkyl bzw. -sulfonylniederalkyl insbesondere $C_3$-$C_5$-Alkenyl-sulfinyl-$C_1$-$C_4$-alkyl bzw. -sulfonyl-$C_1$-$C_4$-alkyl bedeutet.

Niederalkinylthioniederalkyl ist insbesondere $C_3$-$C_5$-Alkinylthio-$C_1$-$C_4$-alkyl, wie 2-Propargylthioethyl oder 3-Propargylthiopropyl, während Niederalkinyl-sulfinylniederalkyl bzw. -sulfonylniederalkyl insbesondere $C_3$-$C_5$-Alkinyl-sulfinyl-$C_1$-$C_4$-alkyl bzw. -sulfonyl-$C_1$-$C_4$-alkyl bedeutet.

Cycloalkyl ist insbesondere $C_3$-$C_7$-Cycloalkyl, d. h. Cyclopropyl, Cyclobutyl, Cyclopentyl, Cyclohexyl oder Cycloheptyl. Bevorzugt sind Cyclopentyl und Cyclohexyl.

Cycloalkenyl ist insbesondere $C_3$-$C_7$-Cycloalkenyl und bedeutet vorzugsweise Cyclopent-2-enyl oder -3-enyl oder Cyclohex-2-enyl oder -3-enyl.

Niederalkoxyniederalkoxyniederalkyl bedeutet insbesondere $C_1$-$C_4$-Alkoxy-$C_1$-$C_4$-alkoxy-$C_1$-$C_4$-alkyl, wie 2-(2-methoxyethoxy)ethyl.

Ausgedehnte pharmakologische Untersuchungen haben ergeben, dass die Verbindungen I und ihre pharmazeutisch verwendbaren Salze z. B. ausgeprägte Angiotensin-II-antagonisierende Eigenschaften aufweisen.

Bekanntlich hat Angiotensin-II starke vasokonstriktorische Eigenschaften und stimuliert ausserdem die Aldosteronsekretion und bewirkt somit eine deutliche Natrium/Wasser- Retention. Die Folge der Angiotensin-II-Aktivität manifestiert sich unter anderem in einer Erhöhung des Blutdrucks. Die Bedeutung von Angiotensin-II-Antagonisten besteht darin, durch kompetitive Hemmung der Bindung von Angiotensin-II an die Rezeptoren die durch Angiotensin-II bewirkten vasokonstriktorischen und die Aldosteronsekretion-stimulierenden Effekte zu unterdrücken.

Die Angiotensin-II-antagonisierenden Eigenschaften der Verbindungen der Formel I und ihrer pharmazeutisch verwendbaren Salze können im Angiotensin-II-Bindungstest erfasst werden. Dabei werden glatte Muskelzellen der Ratte aus homogenisierter Rattenaorta verwendet. Das feste Zentrifugat wird in 50 mM Tris-Puffer (pH 7,4) unter Einsatz von Peptidaseinhibitoren suspendiert. Die Proben werden 60 Minuten bei 25°C mit $^{125}$I-Angiotensin-II (0,175 nM) und einer variierenden Konzentration an Angiotensin-II oder an Testsubstanz inkubiert. Die Inkubation wird dann durch Zugabe von mit eiskaltem Phosphat gepuffertem Kochsalz beendet und es wird durch Whatman GF/F Filter filtriert. Die Filter werden mit einem Gamma-Zähler gezählt. Aus der Dosis-Wirkungs-Kurve werden die $IC_{50}$-Werte bestimmt. Für die Verbindungen der Formel I und ihre pharmazeutisch verwendbaren Salze werden $IC_{50}$-Werte ab etwa 10 nM ermittelt.

Zur Bestimmung der Angiotensin-II induzierten Vasokonstriktion können Untersuchungen an dem isolierten Kaninchen-Aortaring herangezogen werden. Hierzu werden von jeder Brust Aortaringe präpariert und zwischen 2 parallelen Klammern bei einer anfänglich bestehenden Spannung von 2 g fixiert. Anschliessend werden die Ringe bei 37°C in 20 ml eines Gewebebades getaucht und mit einem Gemisch aus 95% $O_2$ und 5% $CO_2$ begast. Die isometrischen Reaktionen werden gemessen. In 20-minütigen Intervallen werden die Ringe abwechselnd mit 10 nM Angiotensin-II (Hypertensin-CIBA) und 5 nM Noradrenalinchlorid stimuliert. Anschliessend werden die Ringe mit ausgewählten Konzentrationen der Testsubstanzen vor der Behandlung mit den Agonisten inkubiert. Die Daten werden mit einem Buxco Digitalcomputer analysiert. Die Konzentrationen, die eine 50%-ige Hemmung der Anfangskontrollwerte bewirken, werden als $IC_{50}$-Werte angegeben. Für die Verbindungen der Formel I und ihre pharmazeutisch verwendbaren Salze werden $IC_{50}$-Werte ab etwa 5

nM bestimmt.

Dass die Verbindungen der Formel I und ihre pharmazeutisch verwendbaren Salze durch Angiotensin-II induzierten Bluthochdruck reduzieren können, kann im Testmodell der normotensiven, narkotisierten Ratte verifiziert werden. Nach Kalibration der Präparationen mit jeweils 0,9% NaCl (1 ml/kg i.v.), Noradrenalin (1 µg/kg i.v.) bzw. Angiotensin-II (0,3 µg/kg i.v.) werden steigende Dosen (3-6) der Testsubstanz durch Bolusinjektion intravenös injiziert, worauf nach jeder Dosis in 5 Minuten-Intervallen Angiotensin-II bzw. Noradrenalin appliziert wird. Der Blutdruck wird direkt in der Halsschlagader gemessen und mit einem on-line Datenerfassungssystem aufgezeichnet (Buxco). Die Spezifität des Angiotensin-II-Antagonismus wird angezeigt durch die selektive Hemmung des von Angiotensin-II, nicht aber des durch Noradrenalin hervorgerufenen Druckeffektes. In diesem Testmodell zeigen die Verbindungen der Formel I und ihre pharmazeutisch verwendbaren Salze ab einer Dosis von etwa 0,3 mg/kg i.v einen hemmenden Effekt.

Auch im Testmodell der renalen hypertensiven Ratte kann die antihypertensive Aktivität der Verbindungen der Formel I und ihrer pharmazeutisch verwendbaren Salze manifestiert werden. Bei männlichen Ratten wird durch Verengung einer renalen Arterie gemäss der Goldblatt-Methode Bluthochdruck erzeugt. Den Ratten werden mittels einer Magensonde Dosen der Testsubstanz verabreicht. Kontrolltiere erhalten ein äquivalentes Volumen an Lösungsmittel. Blutdruck und Herzschlag werden indirekt an wachen Tieren nach der Schwanzklemm-Methode von Gerold et al. [Helv. Physiol. Acta 24 (1966), 58] vor Verabreichung der Testsubstanz bzw. des Lösungsmittels sowie während des Verlaufs der Experimente in Intervallen gemessen. Der ausgeprägte antihypertensive Effekt kann ab einer Dosis von etwa 30 mg/kg p.o. nachgewiesen werden.

Dementsprechend können die Verbindungen der Formel I und ihre pharmazeutisch verwendbaren Salze z.B. als Wirkstoffe in Antihypertensiva verwendet werden, welche z.B. zur Behandlung von Bluthochdruck sowie von Herzinsuffizienz eingesetzt werden. Ein Erfindungsgegenstand ist somit die Verwendung der Verbindungen der Formel I und ihrer pharmazeutisch verwendbaren Salze zur Herstellung von entsprechenden Arzneimitteln und zur therapeutischen Behandlung von Bluthochdruck sowie von Herzinsuffizienz. Bei der Herstellung der Arzneimittel ist auch die gewerbsmässige Herrichtung der Wirksubstanzen eingeschlossen.

Die Erfindung betrifft in erster Linie Verbindungen der Formel I, worin einer der Reste $R_1$ und $R_2$ jeweils gegebenenfalls durch Halogen oder Hydroxy substituiertes Niederalkyl, Niederalkenyl oder Niederalkinyl, jeweils 3- bis 7-gliedriges Cycloalkyl oder Cycloalkenyl, Phenylniederalkyl, Phenylniederalkenyl oder Phenylniederalkinyl bedeutet, der andere der Reste $R_1$ und $R_2$ für die Gruppe Ia steht, in der $Z_1$ für Methylen, Niederalkylen, O, $S(O)_m$ oder N(R) steht, $R_5$ Carboxy, Halogenniederalkansulfonylamino, $SO_3H$, $PO_2H_2$, $PO_3H_2$ oder 5-Tetrazolyl bedeutet und die Ringe A und B unabhängig voneinander gegebenenfalls substituiert sind durch Halogen, durch jeweils gegebenenfalls durch Halogen oder Hydroxy substituiertes Niederalkyl, Niederalkenyl, Niederalkinyl, Niederalkoxyniederalkyl, Niederalkoxyniederalkenyl, Niederalkoxyniederalkinyl, Niederalkenyloxyniederalkyl, Niederalkenyloxyniederalkenyl, Niederalkenyloxyniederalkinyl oder Niederalkoxyniederalkoxyniederalkyl, durch Hydroxy, durch Niederalkoxy, durch Niederalkenyloxy, durch Carboxy, welches gegebenenfalls durch einen Alkohol verestert ist, der sich von Niederalkyl, Niederalkenyl, Niederalkinyl, Niederalkoxyniederalkyl, Niederalkoxyniederalkenyl oder Niederalkoxyniederalkinyl ableitet, durch Carbamoyl, in welchem die Aminogruppe gegebenenfalls durch Niederalkyl, Niederalkenyl, Niederalkinyl, Phenylniederalkyl, Phenylniederalkenyl oder Phenylniederalkinyl unabhängig voneinander mono- oder di-substituiert oder durch Niederalkylen oder Niederalkylenoxyniederalkylen disubstituiert ist, oder durch 5-Tetrazolyl, und entweder $R_3$ Halogen, Niederalkanoyl, gegebenenfalls substituiertes Phenyl, Carboxy, welches gegebenenfalls durch einen Alkohol verestert ist, der sich von Niederalkyl, Niederalkenyl, Niederalkinyl, Niederalkoxyniederalkyl, Niederalkoxyniederalkenyl oder Niederalkoxyniederalkinyl ableitet, Carbamoyl, in welchem die Aminogruppe gegebenenfalls durch Niederalkyl, Niederalkenyl, Niederalkinyl, Phenylniederalkyl, Phenylniederalkenyl oder Phenylniederalkinyl unabhängig voneinander mono- oder di-substituiert ist oder durch Niederalkylen oder Niederalkylenoxyniederalkylen disubstituiert ist, Cyano, $SO_3H$, $PO_2H_2$, $PO_3H_2$, 5-Tetrazolyl, Sulfamoyl, in dem die Aminogruppe gegebenenfalls durch Niederalkyl mono- oder disubstituiert ist, Niederalkansulfonylamino, Halogenniederalkansulfonylamino, Niederalkanoylamino, gegebenenfalls substituiertes Benzoylamino oder gegebenenfalls substituiertes Benzolsulfonylamino bedeutet oder für $-Z_2-R'$ steht, worin $Z_2$ für eine Bindung oder für O, $S(O)_m$ oder N(R) steht und R' Wasserstoff oder jeweils gegebenenfalls durch Hydroxy, Halogen, Amino, Niederalkylenamino, Niederalkylenoxyniederalkylenamino, Niederalkylamino, Niederalkenylamino, Niederalkinylamino, Phenylniederalkylamino, Phenylniederalkenylamino, Phenylniederalkinylamino, Diniederalkylamino, N-Niederalkyl-N-phenylniederalkyl-amino, Di(phenylniederalkyl)amino, Carboxy, welches gegebenenfalls durch einen Alkohol verestert ist, der sich von Niederalkyl, Niederalkenyl, Niederalkinyl, Niederalkoxyniederalkyl, Niederalkoxyniederalkenyl oder Niederalkoxyniederalkinyl ableitet, oder Carbamoyl, in welchem die Aminogruppe gegebenenfalls durch Niederalkyl, Niederalkenyl, Niederalkinyl, Phenylniederalkyl, Phenylniederalkenyl oder Phenylniederalkinyl unabhängig voneinander mono- oder di-substituiert oder durch Niederalkylen oder Niederalkylenoxyniederalkylen disubstituiert ist, substituiertes Niederalkyl, Niederalkenyl,

Niederalkinyl, Niederalkoxyniederalkyl, Niederalkoxyniederalkenyl, Niederalkoxyniederalkinyl, Niederalkenyloxyniederalkyl, Niederalkenyloxyniederalkenyl, Niederalkenyloxyniederalkinyl, Niederalkoxyniederalkoxyniederalkyl, Niederalkylthio-niederalkyl, -niederalkenyl oder -niederalkinyl, Niederalkan-sulfinylniederalkyl oder -sulfonylniederalkyl, Niederalkenyl-thioniederalkyl, -sulfinylniederalkyl oder -sulfonylniederalkyl oder Niederalkinyl-thioniederalkyl, -sulfinylniederalkyl oder -sulfonylniederalkyl bedeutet, und $R_4$ jeweils gegebenenfalls durch Hydroxy, $S(O)_m$-R, jeweils gegebenenfalls substituiertes Phenyl, Phenoxy oder Naphthyloxy, Amino, Niederalkylenamino, Niederalkylenoxyniederalkylenamino, Niederalkylamino, Niederalkenylamino, Niederalkinylamino, Phenylniederalkylamino, Phenylniederalkenylamino, Phenylniederalkynylamino, Diniederalkylamino, N-Niederalkyl-N-phenylniederalkyl-amino, Di(phenylniederalkyl)amino, Carboxy, welches gegebenenfalls durch einen Alkohol verestert ist, der sich von Niederalkyl, Niederalkenyl, Niederalkinyl, Niederalkoxyniederalkyl, Niederalkoxyniederalkenyl oder Niederalkoxyniederalkinyl ableitet, oder Carbamoyl, in welchem die Aminogruppe gegebenenfalls durch Niederalkyl, Niederalkenyl, Niederalkinyl, Phenylniederalkyl, Phenylniederalkenyl oder Phenylniederalkinyl unabhängig voneinander mono- oder di-substituiert oder durch Niederalkylen oder Niederalkylenoxyniederalkylen disubstituiert ist, substituiertes Niederalkyl, Niederalkenyl, Niederalkinyl, Niederalkoxyniederalkyl, Niederalkoxyniederalkenyl, Niederalkoxyniederalkinyl, Niederalkenyloxyniederalkyl, Niederalkenyloxyniederalkenyl, Niederalkenyloxyniederalkinyl, Niederalkoxyniederalkoxyniederalkyl, Niederalkylthio-niederalkyl, -niederalkenyl oder -niederalkinyl, Niederalkan-sulfinylniederalkyl oder -sulfonylniederalkyl, Niederalkenyl-thioniederalkyl, -sulfinylniederalkyl oder -sulfonylniederalkyl oder Niederalkinyl-thioniederalkyl, -sulfinylniederalkyl oder -sulfonylniederalkyl bedeutet, oder $R_3$ und $R_4$ gemeinsam für Methylen oder Niederalkylen stehen, wobei R jeweils Wasserstoff, Niederalkyl, Niederalkenyl oder Niederalkinyl bedeutet, m jeweils für 0, 1 oder 2 steht und aromatische Reste jeweils gegebenenfalls durch Niederalkyl, Niederalkoxy, Halogen, Trifluormethyl und/oder Hydroxy substituiert sind, in freier Form oder in Salzform.

Die Erfindung betrifft insbesondere Verbindungen der Formel I, worin $R_1$ für die Gruppe Ia steht, in der $Z_1$ für Methylen, Niederalkylen, O, $S(O)_m$ oder N(R) steht, $R_5$ Carboxy oder 5-Tetrazolyl bedeutet und die Ringe A und B unabhängig voneinander gegebenenfalls durch Halogen, Niederalkyl, Niederalkoxy, Carboxy, Niederalkoxycarbonyl oder 5-Tetrazolyl substituiert sind, $R_2$ jeweils gegebenenfalls durch Hydroxy oder Halogen substituiertes Niederalkyl oder Niederalkenyl bedeutet und entweder $R_3$ Halogen, Carboxy, welches gegebenenfalls durch einen Alkohol verestert ist, der sich von Niederalkyl oder Niederalkoxyniederalkyl ableitet, Carbamoyl, Cyano, $PO_3H_2$, 5-Tetrazolyl, Niederalkansulfamoyl, Niederalkanoylamino oder Niederalkansulfonylamino bedeutet oder für $-Z_2-R'$ steht, worin $Z_2$ für eine Bindung oder für O, S(O)m oder N(R) steht und R' Wasserstoff oder jeweils gegebenenfalls durch Carboxy, Niederalkoxycarbonyl, Niederalkoxyniederalkoxycarbonyl oder Hydroxy substituiertes Niederalkyl oder Niederalkoxyniederalkyl bedeutet, und $R_4$ Niederalkyl, Niederalkenyl, Niederalkoxyniederalkyl, Carboxyniederalkyl, Niederalkoxycarbonylniederalkyl, Niederalkoxyniederalkoxycarbonylniederalkyl, Carbamoyl-, Niederalkylcarbamoyl- oder Diniederalkylcarbamoyl-niederalkyl, Hydroxyniederalkyl, gegebenenfalls substituiertes Phenoxyniederalkyl, Amino-, Niederalkylamino- oder Diniederalkylamino-niederalkyl oder gegebenenfalls substituiertes Phenylniederalkyl bedeutet, oder $R_3$ und $R_4$ gemeinsam für Methylen oder Niederalkylen stehen, wobei R jeweils Wasserstoff oder Niederalkyl bedeutet, m jeweils 0, 1 oder 2 bedeutet und aromatische Reste jeweils gegebenenfalls durch Niederalkyl, Niederalkoxy, Halogen, Trifluormethyl und/oder Hydroxy substituiert sind, in freier Form oder in Salzform.

Die Erfindung betrifft vor allem Verbindungen der Formel I, worin $R_1$ für die Gruppe Ia steht, in der $Z_1$ für Methylen, Niederalkylen, wie Ethylen, O oder $S(O)_m$ steht, $R_5$ Carboxy oder 5-Tetrazolyl bedeutet und die Ringe A und B unabhängig voneinander gegebenenfalls durch Niederalkyl, wie Methyl, Halogen, insbesondere mit einer Atomnummer bis und mit 35, wie Chlor, oder Niederalkoxy, wie Methoxy, substituiert sind, $R_2$ jeweils gegebenenfalls durch Hydroxy oder Halogen, insbesondere mit einer Atomnummer bis und mit 35, wie Chlor, substituiertes Niederalkyl oder Niederalkenyl, wie n-Propyl, n-Butyl oder Allyl, bedeutet und entweder $R_3$ Halogen, insbesondere mit einer Atomnummer bis und mit 35, wie Chlor, Carboxy, Niederalkoxycarbonyl, wie Methoxy- oder Ethoxycarbonyl, $PO_3H_2$, 5-Tetrazolyl, Niederalkanoylamino, wie Acetylamino, Niederalkansulfonylamino, wie Methansulfonylamino, Wasserstoff, Niederalkyl, wie Methyl, Niederalkoxyniederalkyl, wie 2-Methoxyethyl, Carboxyniederalkyl, wie Carboxymethyl, Niederalkoxycarbonylniederalkyl, wie Ethoxycarbonylmethyl, Hydroxyniederalkyl, wie Hydroxymethyl, Hydroxyniederalkoxyniederalkyl, wie Hydroxyethoxymethyl, Hydroxy, Niederalkoxy, wie Methoxy, Niederalkoxyniederalkoxy, wie 2-Methoxyethoxy, Carboxyniederalkoxy, wie Carboxymethoxy, Niederalkoxycarbonylniederalkoxy, wie Ethoxycarbonylmethoxy, Hydroxyniederalkoxy, wie 2-Hydroxyethoxy, Mercapto, Niederalkylthio, wie Methylthio, Niederalkansulfinyl, wie Methansulfinyl, Niederalkansulfonyl, wie Methansulfonyl, Amino, Niederalkylamino, wie Methylamino, oder Diniederalkylamino, wie Dimethylamino, bedeutet und $R_4$ Niederalkyl, wie Methyl, Ethyl oder n-Butyl, Hydroxyniederalkyl, wie Hydroxymethyl, Carboxyniederalkyl, wie Carboxymethyl, oder Niederalkoxycarbonylniederalkyl, wie Ethoxycarbonylmethyl, bedeutet, oder $R_3$ und $R_4$ gemeinsam für Methylen oder Niederalkylen stehen, wobei m für 0, 1 oder 2 steht und mit "nieder" bezeichnete Teilstrukturen jeweils insbesondere bis und

mit 7, vorzugsweise bis und mit 4, C-Atome umfassen, in freier Form oder in Salzform.

Die Erfindung betrifft besonders Verbindungen der Formel I, worin $R_1$ für die Gruppe Ia steht, in der $Z_1$ für Methylen oder Niederalkylen, wie Ethylen, steht, $R_5$ Carboxy oder 5-Tetrazolyl bedeutet und die Ringe A und B unabhängig voneinander gegebenenfalls durch Niederalkyl, wie Methyl, Halogen, insbesondere mit einer Atomnummer bis und mit 35, wie Chlor, oder Niederalkoxy, wie Methoxy, substituiert sind, $R_2$ Niederalkyl, wie n-Propyl oder n-Butyl, bedeutet, $R_3$ Halogen, insbesondere mit einer Atomnummer bis und mit 35, wie Chlor, Wasserstoff, Niederalkyl, wie Methyl, Niederalkoxyniederalkyl, wie 2-Methoxyethyl, Carboxyniederalkyl, wie Carboxymethyl, Niederalkoxycarbonylniederalkyl, wie Ethoxycarbonylmethyl, Hydroxyniederalkyl, wie Hydroxymethyl, Hydroxyniederalkoxyniederalkyl, wie Hydroxyethoxymethyl, Hydroxy, Niederalkoxy, wie Methoxy, Niederalkoxyniederalkoxy, wie 2-Methoxyethoxy, Carboxyniederalkoxy, wie Carboxymethoxy, Niederalkoxycarbonylniederalkoxy, wie Ethoxycarbonylmethoxy, Hydroxyniederalkoxy, wie 2-Hydroxyethoxy, oder Niederalkoxyniederalkoxy, wie 2-Methoxyniederalkoxy, bedeutet und $R_4$ Niederalkyl, wie Methyl, Ethyl oder n-Butyl, Hydroxyniederalkyl, wie Hydroxymethyl, Carboxyniederalkyl, wie Carboxymethyl, oder Niederalkoxycarbonylniederalkyl, wie Ethoxycarbonylmethyl, bedeutet, wobei mit "nieder" bezeichnete Teilstrukturen jeweils insbesondere bis und mit 7, vorzugsweise bis und mit 4, C-Atome umfassen, in freier Form oder in Salzform.

Die Erfindung betrifft ganz besonders Verbindungen der Formel I, worin $R_1$ für die Gruppe der Formel

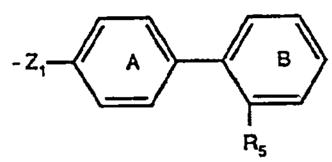

(Ib)

steht, in freier Form oder in Salzform.

Die Erfindung betrifft in ganz besonderer Weise Verbindungen der Formel I, worin $R_1$ für die Gruppe Ib steht, in der $Z_1$ Methylen bedeutet, $R_5$ Carboxy oder insbesondere 5-Tetrazolyl bedeutet und die Ringe A und B unsubstituiert sind, $R_2$ $C_3$-$C_7$-Alkyl, insbesondere $C_3$-$C_5$-Alkyl, wie n-Propyl oder n-Butyl, bedeutet, $R_3$ Wasserstoff oder $C_1$-$C_4$-Alkyl, wie Methyl oder n-Butyl, bedeutet und $R_4$ $C_1$-$C_4$-Alkyl, wie Methyl, Ethyl oder Propyl, Carboxy-$C_1$-$C_4$-alkyl, wie Carboxymethyl, $C_1$-$C_4$-Alkoxycarbonyl-$C_1$-$C_4$-alkyl, wie Ethoxycarbonylmethyl, oder Hydroxy-$C_1$-$C_4$-alkyl, wie Hydroxymethyl, bedeutet, in freier Form oder in Salzform.

Die Erfindung betrifft in allererster Linie Verbindungen der Formel I, worin $R_1$ für die Gruppe Ib steht, in der $Z_1$ Methylen bedeutet, $R_5$ Carboxy oder Tetrazolyl bedeutet und die Ringe A und B unsubstituiert sind, $R_2$ $C_3$-$C_5$-Alkyl, wie n-Propyl oder n-Butyl, bedeutet, $R_3$ Wasserstoff oder $C_1$-$C_4$-Alkyl, wie Methyl oder n-Butyl, bedeutet und $R_4$ $C_1$-$C_4$-Alkyl, wie Methyl, bedeutet, in freier Form oder in Salzform.

Die Erfindung betrifft namentlich die in den Beispielen genannten neuen Verbindungen der Formel I in freier Form oder in Salzform.

Ein weiterer Gegenstand der Erfindung ist ein Verfahren zur Herstellung der Verbindungen der Formel I in freier Form oder in Salzform, z.B. dadurch gekennzeichnet, dass man in einer Verbindung der Formel

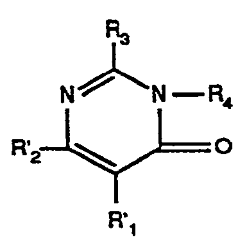

(IIa),

worin einer der Reste $R'_1$ und $R'_2$ für die Gruppe der Formel

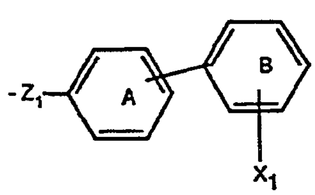

(IIb)

und $X_1$ für einen in $R_5$ überführbaren Rest steht, oder einem Salz davon $X_1$ in $R_5$ überführt und, wenn erwünscht, eine verfahrensgemäss oder auf andere Weise erhältliche Verbindung I in freier Form oder in Salzform in eine andere Verbindung I überführt, ein verfahrensgemäss erhältliches Gemisch von Isomeren auftrennt und das gewünschte Isomere isoliert und/oder eine verfahrensgemäss erhältliche freie Verbindung I in ein Salz oder ein verfahrensgemäss erhältliches Salz einer Verbindung I in die freie Verbindung I oder in ein anderes Salz überführt.

Für Salze von Ausgangsmaterialien gilt das vorstehend für Salze von Verbindungen I Gesagte in analoger Weise.

Die vor- und nachstehend beschriebenen Umsetzungen werden in an sich bekannter Weise durchgeführt, z.B. in Ab- oder üblicherweise in Anwesenheit eines geeigneten Lösungs- oder Verdünnungsmittels oder eines Gemisches derselben, wobei man je nach Bedarf unter Kühlen, bei Raumtemperatur oder unter Erwärmen, z.B. in einem Temperaturbereich von etwa -80°C bis zur Siedetemperatur des Reaktionsmediums, vorzugsweise von etwa -10° bis etwa +200°C, und, falls erforderlich, in einem geschlossenen Gefäss, unter Druck, in einer Inertgasatmosphäre und/oder unter wasserfreien Bedingungen arbeitet.

In die Variable $R_5$ überführbare Reste $X_1$ sind beispielsweise Cyano, Mercapto, Halogen, die Gruppe $-N_2^+ A^-$, in der $A^-$ ein von einer Säure abgeleitetes Anion bedeutet, Amino, funktionelle Derivate von COOH, $SO_3H$, $PO_3H_2$ und $PO_2H_2$ und N-geschütztes 5-Tetrazolyl.

Funktionell abgewandeltes Carboxy bedeutet beispielsweise Cyano oder verestertes oder amidiertes Carboxy.

In 5-Tetrazolyl $R_5$ überführbare Reste $X_1$ sind beispielsweise Cyano und N-geschütztes 5-Tetrazolyl.

Zur Herstellung von Verbindungen der Formel I, worin $R_5$ 5-Tetrazolyl bedeutet, geht man beispielsweise von Ausgangsmaterial der Formel IIa aus, worin $X_1$ Cyano bedeutet, und setzt dieses mit einem Azid, z. B. mit $HN_3$ oder insbesondere einem Salz, wie Alkalimetallsalz, davon oder mit einem Organozinnazid, wie Triniederalkyl- oder Triaryl-zinnazid, um. Bevorzugte Azide sind beispielsweise Natrium- und Kaliumazid sowie Tri-$C_1$-$C_4$-alkyl-, z.B. Triethyl- oder Tributyl-zinnazid, und Triphenylzinnazid.

Als Schutzgruppen von N-geschütztem 5-Tetrazolyl kommen die üblicherweise in der Tetrazolchemie verwendeten Schutzgruppen in Frage, insbesondere Triphenylmethyl, gegebenenfalls, z.B. durch Nitro, substituiertes Benzyl, wie 4-Nitrobenzyl, Niederalkoxymethyl, wie Methoxy- oder Ethoxymethyl, Niederalkylthiomethyl, wie Methylthiomethyl, sowie 2-Cyanoethyl, ferner Niederalkoxyniederalkoxymethyl, wie 2-Methoxyethoxymethyl, Benzyloxymethyl sowie Phenacyl. Die Abspaltung der Schutzgruppen erfolgt in Anlehnung an bekannte Methoden. So wird z.B. Triphenylmethyl üblicherweise durch Hydrolyse, insbesondere in Gegenwart einer Säure, oder Hydrogenolyse in Gegenwart eines Hydrierungskatalysators, 4-Nitrobenzyl z.B. durch Hydrogenolyse in Gegenwart eines Hydrierungskatalysators, Methoxy- oder Ethoxy-methyl z.B. durch Behandeln mit einem Triniederalkyl-, wie Triethyl- oder Tributyl-zinn-bromid, Methylthiomethyl z.B. durch Behandeln mit Trifluoressigsäure, 2-Cyanoethyl z.B. durch Hydrolyse, beispielsweise mit Natronlauge, 2-Methoxyethoxymethyl z.B. durch Hydrolyse, z.B. mit Salzsäure, und Benzyloxymethyl und Phenacyl z.B. durch Hydrogenolyse in Gegenwart eines Hydrierungskatalysators abgespalten.

Ein in $SO_3H$ $R_5$ überführbarer Rest $X_1$ ist beispielsweise die Mercaptogruppe. Eine solche Gruppe aufweisende Ausgangsverbindungen der Formel IIa werden beispielsweise durch an sich bekannte Oxidationsverfahren zu solchen Verbindungen der Formel I oxidiert, worin $R_5$ $SO_3H$ ist. Als Oxidationsmittel kommen beispielsweise anorganische Persäuren, wie Persäuren von Mineralsäuren, z.B. Periodsäure oder Perschwefelsäure, organische Persäuren, wie Percarbon- oder Persulfon-säuren, z.B. Perameisen-, Peressig-, Trifluorperessig- oder Perbenzoe-säure oder p-Toluolpersulfonsäure, oder Gemische aus Wasserstoffperoxid und Säuren, z.B. Gemische aus Wasserstoffperoxid und Essigsäure, in Betracht. Häufig führt man die Oxidation in Gegenwart von geeigneten Katalysatoren durch, wobei als Katalysatoren geeignete Säuren, wie gegebenenfalls substituierte Carbonsäuren, z.B. Essigsäure oder Trifluoressigsäure, oder Übergangsmetalloxide, wie Oxide von Elementen der VI. Nebengruppe, z.B. Molybdän- oder Wolfram-oxid, zu nennen sind. Die Oxidation wird unter milden Bedingungen, z.B. bei Temperaturen von etwa -50° bis etwa +100°C, durchgeführt.

Unter einer in $PO_3H_2$ $R_5$ überführbaren Gruppe ist beispielsweise eine Gruppe $-N_2^+A^-$ zu verstehen, wobei $A^-$ für ein Anion einer Säure, wie Mineralsäure, steht. Entsprechende Diazoniumverbindungen werden beispielsweise in an sich bekannter Weise mit einem P(III)-Halogenid, wie $PCl_3$ oder $PBr_3$, umgesetzt und hydrolytisch aufgearbeitet, wobei solche Verbindungen der Formel I erhältlich sind, worin $R_5PO_3H_2$ ist.

Verbindungen I, worin $R_5$ $PO_2H_2$ ist, erhält man z. B. durch in üblicher Weise erfolgende Umwandlung von $X_1$ in einer Verbindung IIa, worin $X_1$ ein funktionelles Derivat von $PO_2H_2$ ist, in $PO_2H_2$.

Als in Halogenalkansulfonylamino $R_5$ überführbarer Rest $X_1$ kommt beispielsweise Amino in Frage. Zur Herstellung von Verbindungen der Formel I, worin $R_5$ Halogenalkansulfonylamino bedeutet, setzt man beispielsweise entsprechende Aniline mit einer üblicherweise reaktionsfähig veresterten Halogenalkansulfonsäure um, wobei gegebenenfalls in Gegenwart einer Base gearbeitet wird. Die bevorzugte reaktionsfähig

veresterte Halogenalkansulfonsäure ist das entsprechende Halogenid, wie Chlorid oder Bromid.

Ein in COOH $R_5$ überführbarer Rest $X_1$ steht beispielsweise für funktionell abgewandeltes Carboxy, wie Cyano, verestertes oder amidiertes Carboxy, Hydroxymethyl oder Formyl.

Verestertes Carboxy ist beispielsweise mit einem gegebenenfalls substituierten aliphatischen, cycloaliphatischen oder aromatischen Alkohol verestertes Carboxy. Ein aliphatischer Alkohol ist beispielsweise ein Niederalkanol, wie Methanol, Ethanol, n-Propanol, Isopropanol, n-Butanol, sek.-Butanol oder tert.-Butanol, während als cycloaliphatischer Alkohol beispielsweise ein 3- bis 8-gliedriges Cycloalkanol, wie Cyclo-pentanol, -hexanol oder -heptanol, in Frage kommt. Ein aromatischer Alkohol ist beispielsweise ein Phenol oder ein heterocyclischer Alkohol, welche jeweils substituiert sein können, insbesondere Hydroxypyridin, z.B. 2-, 3- oder 4-Hydroxypyridin.

Amidiertes Carboxy ist beispielsweise Carbamoyl, durch Hydroxy, Amino oder gegebenenfalls substituiertes Phenyl monosubstituiertes, durch Niederalkyl mono- oder disubstituiertes oder durch 4- bis 7-gliedriges Alkylen oder 3-Aza-, 3-Niederalkylaza-, 3-Oxa- oder 3-Thiaalkylen disubstituiertes Carbamoyl. Als Beispiele sind Carbamoyl, N-Mono- oder N,N-Diniederalkylcarbamoyl, wie N-Methyl-, N-Ethyl-, N,N-Dimethyl-, N,N-Diethyl- und N,N-Dipropyl-carbamoyl, Pyrrolidino- und Piperidinocarbonyl, Morpholino-, Piperazino-, 4-Methylpiperazino- und Thiomorpholino-carbonyl, Anilinocarbonyl und durch Niederalkyl, Niederalkoxy und/oder Halogen substituiertes Anilinocarbonyl zu nennen.

Bevorzugtes funktionell abgewandeltes Carboxy ist beispielsweise Niederalkoxycarbonyl, wie Methoxy- oder Ethoxycarbonyl, und Cyano.

Verbindungen der Formel I, worin $R_5$ Carboxy ist, können beispielsweise ausgehend von Verbindungen der Formel IIa, worin $X_1$ Cyano oder verestertes oder amidiertes Carboxy bedeutet, durch Hydrolyse, insbesondere in Gegenwart einer Base, oder, ausgehend von Verbindungen der Formel IIa, worin $X_1$ Hydroxymethyl oder Formyl bedeutet, durch Oxidation hergestellt werden. Die Oxidation erfolgt beispielsweise in einem inerten Lösungsmittel, wie in einer Niederalkancarbonsäure, z.B. Essigsäure, einem Keton, z.B. Aceton, einem Ether, z.B. Tetrahydrofuran, einem heterocyclischen Aromaten, z.B. Pyridin, oder in Wasser, oder in einem Gemisch davon, erforderlichenfalls unter Kühlen oder Erwärmen, z.B. in einem Temperaturbereich von etwa 0° bis etwa +150°C. Als Oxidationsmittel kommen beispielsweise oxidierende Übergangsmetallverbindungen, insbesondere solche mit Elementen der I., VI. oder VII. Nebengruppe, in Frage. Als Beispiele seien genannt: Silberverbindungen, wie Silber-nitrat, -oxid und -picolinat, Chromverbindungen, wie Chromtrioxid und Kaliumdichromat, und Manganverbindungen, wie Kalium-, Tetrabutylammonium- und Benzyltriethylammonium-permanganat. Weitere Oxidationsmittel sind beispielsweise geeignete Verbindungen mit Elementen der IV. Hauptgruppe, wie Bleidioxid, oder Halogen-Sauerstoff-Verbindungen, wie Natriumiodat oder Kaliumperiodat.

Das Ausgangsmaterial IIa ist beispielsweise zugänglich, indem man von einer Verbindung der Formel

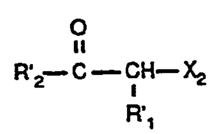

(IIc),

worin $X_2$ gegebenenfalls funktionell abgewandeltes Carboxy bedeutet, deren Herstellung in an sich bekannter Weise erfolgt, ausgeht und diese mit einer Verbindung der Formel

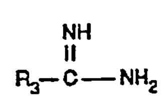

(IId)

oder einem Salz davon umsetzt.

Die Umsetzung wird erforderlichenfalls in Gegenwart einer Base durchgeführt.

Als Basen kommen beispielsweise Alkalimetall-hydroxide, -hydride, -amide, -alkanolate, -carbonate, -triphenylmethylide, -diniederalkylamide, -aminoalkylamide oder -niederalkylsilylamide, Naphthalinamine, Niederalkylamine, basische Heterocyclen, Ammoniumhydroxide, sowie carbocyclische Amine in Frage. Beispielhaft seien Natriumhydroxid, -hydrid, -amid, Kalium-tert-butylat, -carbonat, Lithium-triphenylmethylid, -diisopropylamid, Kalium-3-(aminopropyl)-amid, -bis-(trimethylsilyl)-amid, Dimethylaminonaphthalin, Di- oder Triethylamin, oder Ethyl-diisopropylamin, N-Methyl-piperidin, Pyridin, Benzyltrimethyl-ammoniumhydroxid, 1,5-Diazabicyclo[4.3.0]non-5-en (DBN) sowie 1,8-Diaza-bicyclo[5.4.0]undec-7-en (DBU) genannt.

In Verbindungen IIa, worin $R_4$ Wasserstoff ist, kann in an sich bekannter Weise der Rest $R_4$ durch übliche

Alkylierung mit entsprechenden Alkylierungsreagentien eingeführt werden.

Verbindungen IIa können ebenfalls hergestellt werden, indem man von Verbindungen IIc ausgeht und diese mit einer Verbindung der Formel $R_3$-C(=NH)-NH-$R_4$ (IIe) oder einem Salz, insbesondere Säureadditionssalz, davon in Gegenwart einer Base umsetzt. Verbindungen IIe wiederum sind beispielsweise zugänglich durch Umsetzung von Verbindungen IId oder einem Salz, insbesondere Säureadditionssalz, davon mit einem Amin der Formel $R_4$-$NH_2$ (IIf) oder einem Salz, insbesondere Säureadditionssalz, davon.

Eine verfahrensgemäss oder auf andere Weise erhältliche Verbindung I kann in an sich bekannter Weise in eine andere Verbindung I überführt werden.

Eine Hydroxy aufweisende Verbindung I kann z. B. nach an sich bekannten Methoden verethert werden. Die Veretherung kann z.B. mit einem Alkohol, wie einem gegebenenfalls substituierten Niederalkanol, oder einem reaktionsfähigen Ester desselben erfolgen. Als reaktionsfähige Ester der gewünschten Alkohole kommen beispielsweise solche mit starken anorganischen oder organischen Säuren in Frage, wie entsprechende Halogenide, Sulfate, Niederalkansulfonate oder gegebenenfalls substituierte Benzolsulfonate, z.B. Chloride, Bromide, Iodide oder Methan-, Benzol- oder p-Toluol-sulfonate, in Betracht. Die Veretherung kann z.B. in Gegenwart einer Base, z. B. eines Alkalimetall-hydrids, -hydroxids oder -carbonats oder eines basischen Amins, erfolgen. Umgekehrt können entsprechende Ether, wie Niederalkoxyverbindungen, z.B. mittels starker Säuren, wie Mineralsäuren, z.B. Brom- oder Iod-wasserstoffsäure, die vorteilhaft in Form von Pyridiniumhalogeniden vorliegen können, oder mittels Lewissäuren, z.B. Halogeniden von Elementen der III. Hauptgruppe oder der entsprechenden Nebengruppen, gespalten werden. Diese Umsetzungen können, falls erforderlich, unter Kühlen oder Erwärmen, z.B. in einem Temperaturbereich von etwa -20° bis etwa +100°C, in An- oder Abwesenheit eines Lösungs- oder Verdünnungsmittels, unter Inertgas und/oder unter Druck und gegebenenfalls in einem geschlossenen Gefäss durchgeführt werden.

Hydroxymethylgruppen aufweisende Verbindungen I können beispielsweise ausgehend von entsprechenden Carboxy oder verestertes Carboxy aufweisenden Verbindungen hergestellt werden, indem entsprechende Verbindungen in an sich bekannter Weise reduziert werden, z.B. durch Hydrierung mit Wasserstoff in Gegenwart eines der nachstehend genannten Hydrierungskatalysatoren oder insbesondere durch Reduktion mit einem gegebenenfalls komplexen Hydrid, wie einem Hydrid gebildet aus einem Element der 1. und 3. Hauptgruppe des Periodensystems der Elemente, z.B. einem Boranat oder Alanat, beispielsweise Natriumborhydrid, Lithiumaluminiumhydrid oder Diisobutylaluminiumhydrid, ferner Diboran.

Falls ein aromatischer Strukturbestandteil durch Niederalkylthio substituiert ist, kann man dieses auf übliche Weise zu entsprechendem Niederalkan-sulfinyl oder -sulfonyl oxidieren. Als geeignetes Oxidationsmittel für die Oxidation zur Sulfoxidstufe kommen beispielsweise anorganische Persäuren, wie Persäuren von Mineralsäuren, z.B. Periodsäure oder Perschwefelsäure, organische Persäuren, wie Percarbon- oder Persulfonsäuren, z.B. Perameisen-, Peressig-, Trifluorperessig-, Perbenzoe- oder p-Toluolpersulfon-säure, oder Gemische aus Wasserstoffperoxid und Säuren, z.B. Gemische aus Wasserstoffperoxid und Essigsäure, in Betracht. Häufig führt man die Oxidation in Gegenwart von geeigneten Katalysatoren durch, wobei als Katalysatoren geeignete Säuren, wie gegebenenfalls substituierte Carbonsäuren, z.B. Essigsäure oder Trifluoressigsäure, oder Übergangsmetalloxide, wie Oxide von Elementen der VI. Nebengruppe, z.B. Molybdän- oder Wolfram-oxid, zu nennen sind. Die Oxidation wird unter milden Bedingungen, z.B. bei Temperaturen von etwa -50° bis etwa +100°C, durchgeführt. Die weitere Oxidation zur Sulfonstufe kann man mit Distickstofftetroxid als Katalysator in Gegenwart von Sauerstoff bei tiefen Temperaturen entsprechend durchführen, ebenso wie die direkte Oxidation von Niederalkylthio zu Niederalkansulfonyl. Jedoch setzt man hierbei üblicherweise das Oxidationsmittel im Überschuss ein.

Weist eine der Variablen Amino auf, können entsprechende Verbindungen I in an sich bekannter Weise N-(ar)alkyliert werden ; ebenso können Carbamoyl bzw. Carbamoyl aufweisende Reste N-(ar)alkyliert werden. Die (Ar-)Alkylierung erfolgt z.B. mit einem (Aryl-)$C_1$-$C_7$-Alkyl-halogenid, z.B. -bromid oder -iodid, (Aryl-)$C_1$-$C_7$-Alkansulfonat, z.B. mit Methansulfonat oder p-Toluolsulfonat, oder einem Di-$C_1$-$C_7$-alkylsulfat, z.B. Dimethylsulfat, vorzugsweise unter basischen Bedingungen, wie in Gegenwart von Natronlauge oder Kalilauge, und vorteilhaft in Gegenwart eines Phasentransfer-Katalysators, wie von Tetrabutylammoniumbromid oder Benzyltrimethylammoniumchlorid, wobei indes stärker basische Kondensationsmittel, wie Alkalimetall-amide, -hydride oder -alkoholate, z.B. Natriumamid, Natriumhydrid oder Natriumethanolat, erforderlich sein können.

In Verbindungen der Formel I, die als Substituenten eine veresterte oder amidierte Carboxygruppe aufweisen, kann man eine solche Gruppe, z.B. mittels Hydrolyse, z.B. in Gegenwart eines basischen Mittels oder eines sauren Mittels, wie einer Mineralsäure, in eine freie Carboxygruppe überführen.

Ferner kann man in Verbindungen der Formel I, die als Substituenten eine Carboxygruppe aufweisen (insbesondere, sofern $R_5$ von Carboxy verschieden ist), diese, z.B. durch Behandeln mit einem Alkohol, wie einem Niederalkanol, in Gegenwart eines geeigneten Veresterungsmittels, wie eines sauren Reagens, z.B. einer anorganischen oder organischen Säure oder einer Lewissäure, z.B. von Zinkchlorid, oder eines wasserbindenden Kondensationsmittels, z.B. eines Carbodiimids, wie von N,N'-Dicyclohexylcarbodiimid, oder durch Behandeln mit einem Diazoreagens, wie mit einem Diazoniederalkan, z.B. Diazomethan, in eine veresterte Carboxygruppe überführen. Diese kann man auch erhalten, wenn man Verbindungen der Formel I, worin die Carboxygruppe in freier Form oder in Salz-, wie Ammonium- oder Metall-, z.B. Alkalimetall-, wie Natrium- oder Kalium-salzform vorliegt, mit einem $C_1$-$C_7$-Alkylhalogenid, z.B. Methyl- oder Ethyl-bromid oder -iodid, oder einem organischen Sulfonsäureester, wie einem entsprechenden $C_1$-$C_7$-Alkylester, z.B. Methansulfonsäure- oder p-Toluolsulfonsäure-methylester oder -ethylester, behandelt.

Verbindungen der Formel I, die als Substituenten eine veresterte Carboxygruppe aufweisen, kann man durch Umesterung, z.B. durch Behandeln mit einem Alkohol, üblicherwiese mit einem höheren als dem der veresterten Carboxygruppe im Ausgangsmaterial entsprechenden Alkohol, in Gegenwart eines geeigneten Umesterungsmittels, wie eines basischen Mittels, z.B. eines Alkalimetall-$C_1$-$C_7$-alkanoats, -$C_1$-$C_7$-alkanolats oder -cyanids, wie von Natrium-acetat, -methanolat, -ethanolat, -tert.-butanolat oder -cyanid, oder eines geeigneten sauren Mittels, gegebenenfalls unter Entfernung des entstehenden Alkohols, z.B. durch Destillation, in andere Esterverbindungen der Formel I umwandeln. Man kann auch von entsprechenden, sogenannten aktivierten Estern der Formel I ausgehen, die als Substituenten eine aktivierte veresterte Carboxygruppe aufweisen (siehe unten), und diese durch Behandeln mit einem $C_1$-$C_7$-Alkanol in einen anderen Ester umwandeln.

Man kann in Verbindungen der Formel I, die als Substituenten die Carboxygruppe enthalten, diese auch zuerst in ein reaktionsfähiges Derivat, wie ein Anhydrid (auch ein gemischtes Anhydrid), ein Säure-halogenid, z.B. -chlorid (z.B. durch Behandeln mit einem Thionyl-halogenid, z.B. -chlorid), ein Anhydrid mit einem Ameisensäure-ester, z.B. -$C_1$-$C_7$-alkylester (z.B. durch Behandeln eines Salzes, wie eines Ammonium- oder Alkalimetallsalzes, mit einem Halogen-, wie Chlor-ameisensäureester, wie $C_1$-$C_7$-Alkyl- ester), oder einen aktivierten Ester, wie Cyanmethyl-, Nitrophenyl-, z.B. 4-Nitro-phenyl-, oder Polyhalogenphenyl-, z.B. Pentachlorphenyl-ester (z.B. durch Behandeln mit einer entsprechenden Hydroxyverbindung in Gegenwart eines geeigneten Kondensationsmittels, wie von N,N'-Dicyclohexylcarbodiimid) überführen, und ein solches reaktionsfähiges Derivat dann mit einem Amin umsetzen und so zu Amidverbindungen der Formel I gelangen, die als Substituenten eine amidierte Carboxygruppe aufweisen. Dabei kann man diese direkt oder über Zwischenverbindungen erhalten ; so kann man z.B. einen aktivierten Ester, wie einen 4-Nitrophenylester, einer Verbindung der Formel I mit einer Carboxygruppe zuerst mit einem 1-unsubstituierten Imidazol umsetzen und die so entstandene 1-Imidazolylcarbonylverbindung mit einem Amin in Reaktion bringen. Man kann aber auch andere, nicht-aktivierte Ester, wie $C_1$-$C_7$-Alkylester, von Verbindungen der Formel I mit Aminen zur Reaktion bringen.

Weist ein aromatischer Ring als Substituenten ein Wasserstoffatom auf, so kann dieses mit Hilfe eines Halogenierungsmittels in üblicher Weise durch ein Halogenatom ersetzt werden, z.B. mit Brom, Hypobromsäure, einem Acylhypobromit oder einer anderen organischen Bromverbindung, z.B. N-Bromsuccinimid, N-Bromacetamid, N-Bromphthalimid, Pyridiniumperbromid, Dioxandibromid, 1,3-Dibrom-5,5-dimethylydantoin oder 2,4,4,6-Tetrabrom-2,5-cyclohexandien-1-on, durch Brom oder mit elementarem Chlor, z.B. in einem halogenierten Kohlenwasserstoff, wie Chloroform, und unter Kühlen, z.B. bis auf etwa -10°C, durch Chlor.

Enthält ein aromatischer Ring eine Aminogruppe, so kann diese in üblicher Weise diazotiert werden, z.B. durch Behandeln mit einem Nitrit, z.B. Natriumnitrit, in Gegenwart einer geeigneten Protonsäure, z.B. einer Mineralsäure, wobei die Reaktionstemperatur vorteilhaft unter etwa 5°C gehalten wird. Die so erhältliche, in Salzform vorliegende Diazoniumgruppe kann man nach üblichen Verfahren beispielsweise wie folgt substituieren : durch die Hydroxygruppe analog der Phenolverkochung in Gegenwart von Wasser ; durch eine Alkoxygruppe durch Behandeln mit einem entsprechenden Alkohol, wobei Energie zugeführt werden muss ; durch das Fluoratom analog der Schiemann-Reaktion bei der Thermolyse von entsprechenden Diazoniumtetrafluorboraten ; oder durch Chlor, Brom, Iod oder die Cyanogruppe analog der Sandmeyer-Reaktion durch Umsetzung mit entsprechenden Cu(I)-Salzen, zunächst unter Kühlen, z.B. auf unter etwa 5°C, und anschliessendem Erhitzen, z.B. auf etwa 60° bis etwa 150°C.

Enthalten die Verbindungen der Formel I ungesättigte Reste, wie Niederalkenyl oder Niederalkinylgruppierungen, können diese in an sich bekannter Weise in gesättigte Reste überführt werden. So erfolgt beispielsweise die Hydrierung von Mehrfachbindungen durch katalytische Hydrierung in Gegenwart von Hydrierungskatalysatoren, wobei hierfür z.B. Nickel, wie Raney-Nickel, sowie Edelmetalle oder deren Derivate, z.B. Oxide, geeignet sind, wie Palladium oder Platinoxid, die gegebenenfalls auf Trägermaterialien, z.B. auf Kohle oder Calciumcarbonat, aufgezogen sein können. Die Hydrierung kann vorzugsweise bei Drucken zwischen etwa 1 und etwa 100 at und bei Temperaturen zwischen etwa -80° und etwa +200°C, vor allem zwischen Raumtemperatur und etwa 100°C, durchgeführt werden. Die Reaktion erfolgt zweckmässig in einem Lösungsmittel, wie Wasser, einem Niederalkanol, z.B. Ethanol, Isopropanol oder n-Butanol, einem Ether, z.B. Dioxan, oder einer Niederalkancarbonsäure, z.B. Essigsäure.

Weiterhin kann in Verbindungen I, worin z.B. einer der Reste $R_1$, $R_2$, $R_3$ und $R_4$ Halogen, wie Chlor, bedeu-

tet, Halogen durch Umsetzung mit einem gegebenenfalls substituierten Amin, einem Alkohol oder Mercaptan ausgetauscht werden.

Die Erfindung betrifft insbesondere die in den Beispielen beschriebenen Verfahren.

Salze von Verbindungen der Formel I können in an sich bekannter Weise hergestellt werden. So erhält man beispielsweise Säureadditionssalze von Verbindungen der Formel I durch Behandeln mit einer geeigneten Säure oder einem geeigneten Ionenaustauscherreagens. Salze von Verbindungen I können in üblicher Weise in die freien Verbindungen I überführt werden, Säureadditionssalze z.B. durch Behandeln mit einem geeigneten basischen Mittel oder einem geeigneten Ionenaustauscherreagens.

Salze von Verbindungen I können in an sich bekannter Weise in andere Salze von Verbindungen I umgewandelt werden.

Je nach Verfahrensweise bzw. Reaktionsbedingungen können die Verbindungen I mit salzbildenden, insbesondere basischen Eigenschaften, in freier Form oder in Form von Salzen erhalten werden.

Infolge der engen Beziehung zwischen der Verbindung I in freier Form und in Form ihrer Salze sind im Vorausgegangenen und nachfolgend unter der freien Verbindung I bzw. ihren Salzen sinn- und zweckgemäss gegebenenfalls auch die entsprechenden Salze bzw. die freie Verbindung I zu verstehen.

Die Verbindungen I einschliesslich ihrer Salze von salzbildenden Verbindungen können auch in Form ihrer Hydrate erhalten werden und/oder andere, z. B. zur Kristallisation verwendete, Lösungsmittel einschliessen.

Die Verbindungen I und ihre Salze können, je nach der Wahl der Ausgangsstoffe und Arbeitsweisen, in Form eines der möglichen Isomeren oder als Gemisch derselben, z.B. je nach Anzahl, absoluter und relativer Konfiguration der asymmetrischen Kohlenstoffatome als reine Isomere, wie Antipoden und/oder Diastereomere, oder als Isomerengemische, wie Enantiomerengemische, z. B. Racemate, Diastereomerengemische oder Racematgemische, vorliegen.

Erhaltene Diastereomerengemische und Racematgemische können auf Grund der physikalisch-chemischen Unterschiede der Bestandteile in bekannter Weise in die reinen Diastereomeren oder Racemate aufgetrennt werden, beispielsweise durch fraktionierte Kristallisation. Erhaltene Enantiomerengemische, wie Racemate, lassen sich nach bekannten Methoden in die optischen Antipoden zerlegen, beispielsweise durch Umkristallisation aus einem optisch aktiven Lösungsmittel, Chromatographie an chiralen Adsorbentien, mit Hilfe von geeigneten Mikroorganismen, durch Spaltung mit spezifischen, immobilisierten Enzymen, über die Bildung von Einschlussverbindungen, z.B. unter Verwendung chiraler Kronenether, wobei nur ein Enantiomeres komplexiert wird, oder durch Überführung in diastereomere Salze, z.B. durch Umsetzung eines basischen Endstoffracemats mit einer optisch aktiven Säure, wie Carbonsäure, z.B. Wein- oder Äpfelsäure, oder Sulfonsäure, z.B. Camphersulfonsäure, und Trennung des auf diese Weise erhaltenen Diastereomerengemisches, z.B. auf Grund ihrer verschiedenen Löslichkeiten, in die Diastereomeren, aus denen das gewünschte Enantiomere durch Einwirkung geeigneter Mittel freigesetzt werden kann. Vorteilhaft isoliert man das wirksamere Enantiomere.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates bzw. Salzes und/oder seiner Racemate bzw. Antipoden verwendet oder insbesondere unter den Reaktionsbedingungen bildet.

Beim Verfahren der vorliegenden Erfindung werden vorzugsweise solche Ausgangsstoffe und Zwischenprodukte verwendet, welche zu den eingangs als besonders wertvoll geschilderten Verbindungen I führen. Neue Ausgangsstoffe und Zwischenprodukte für die Herstellung der Verbindungen I, ihre Verwendung und ein Verfahren zu ihrer Herstellung bilden ebenfalls einen Gegenstand der Erfindung, wobei die Variablen $R_1$, $R_2$, $R_3$ und $R_4$ die für die Verbindungen I angegebenen Bedeutungen haben.

Die Verbindungen I und ihre pharmazeutisch verwendbaren Salze können, vorzugsweise in Form von pharmazeutisch verwendbaren Zubereitungen, in einem Verfahren zur prophylaktischen und/oder therapeutischen Behandlung des tierischen oder menschlichen Körpers, insbesondere als Antihypertensiva, verwendet werden.

Die Erfindung betrifft daher gleichfalls pharmazeutische Präparate, die eine Verbindung I in freier Form oder in Form eines pharmazeutisch verwendbaren Salzes als Wirkstoff enthalten, sowie ein Verfahren zu ihrer Herstellung. Bei diesen pharmazeutischen Präparaten handelt es sich um solche zur enteralen, wie oralen, ferner rektalen oder parenteralen Verabreichung an Warmblüter, wobei der pharmakologische Wirkstoff allein oder zusammen mit üblichen pharmazeutischen Hilfsstoffen enthalten ist. Die pharmazeutischen Präparate enthalten z.B. von etwa 0,1 bis bis 100%, vorzugsweise von etwa 1% bis etwa 60%, des Wirkstoffs. Pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren, hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw.

Granulat, wenn erwünscht oder notwendig nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragantgummi, Methylcellulose und/oder Polyvinylpyrrolidon, und, wenn erwünscht, Sprengmittel, wie die obengenannten Stärken, ferner Carboxymethylstärke, quervernetztes Polyvinylpyrrolidon, Agar oder Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls Magensaftresistenten Überzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine sowie weiche, geschlossene Kapseln aus Gelatine und einem Weichmacher, wie Glycerin oder Sorbitol. Die Steckkapseln können den Wirkstoff in Form eines Granulates, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Ölen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole und höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyethylenglykole und Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Öle, z.B. Sesamöl, oder synthetische Fettsäureester, z.B. Ethyloleat oder Triglyceride, verwendet, oder wässrige Injektionssuspensionen, welche viskositätserhöhende Stoffe, z.B. Natriumcarboxymethylcellulose, Sorbit und/oder Dextran, und gegebenenfalls auch Stabilisatoren enthalten.

Die Dosierung des Wirkstoffes kann von verschiedenen Faktoren, wie Applikationsweise, Warmblüter-Spezies, Alter und/oder individuellem Zustand, abhängen. Im Normalfall ist für einen etwa 75 kg schweren Patienten bei oraler Applikation eine ungefähre Tagesdosis von etwa 10 mg bis etwa 250 mg zu veranschlagen.

Die nachfolgenden Beispiele illustrieren die oben beschriebene Erfindung ; sie sollen jedoch diese in ihrem Umfang in keiner Weise einschränken. Temperaturen sind in Grad Celsius angegeben.

Beispiel 1 : Ein Gemisch aus 870 mg (2,34 mmol) 4-(n-Butyl)-5-(2′-cyanobiphenyl-4-ylmethyl)- 1,2-dimethyl-6-oxo- 1,6-dihydro-pyrimidin, 1,55 g (4,68 mmol) Tributylzinnazid und 30 ml o-Xylol wird unter Rühren 24 Stunden unter Rückfluss erhitzt. Anschliessend wird das Reaktionsgemisch im Vakuum eingedampft, der Rückstand in 50 ml eines Dichlormethan/Methanol/Ammoniak-Gemisches (5 :3 :1) aufgenommen und dieses Gemisch 30 Minuten gerührt. Nach erneutem Eindampfen im Vakuum wird der Rückstand mittels Flash-Chromatographie [Kieselgel 60 (40-63 μm), Dichlormethan/Methanol/Ammoniak (80 :10 :1)] aufgetrennt. Man erhält so das 4-(n-Butyl)- 1,2-dimethyl-6-oxo-5-[2′(1H-tetrazol-5-yl)biphenyl-4-ylmethyl]- 1,6-dihydro-pyrimidin, in Form eines amorphen Festkörpers, der aus Acetonitril kristallisiert [Smp. : 165-169° (Zersetzung)].

Das Ausgangsmaterial kann z. B. wie folgt hergestellt werden :

a) Zu einer Lösung von 1,0 g (2,8 mmol) 6-(n-Butyl)-5-(2′-cyanobiphenyl-4-ylmethyl)-4-hydroxy-2-methylpyrimidin in 20 ml N,N-Dimethylformamid werden bei Raumtemperatur 84 mg (2,8 mmol) Natriumhydrid (80 % in Weissoel) zugegeben. Nach beendeter Zugabe wird das Reaktionsgemisch 30 Minuten nachgerührt. Anschliessend werden 0,23 ml (3,7 mmol) Methyliodid zugetropft. Das Reaktionsgemisch wird sodann 3 Stunden bei Raumtemperatur gerührt und anschliessend im Vakuum eingedampft. Der Rückstand wird zwischen Essigsäureethylester und Wasser verteilt und die organische Phase mit Wasser und gesättigter Natriumchloridlösung gewaschen, über $Na_2SO_4$ getrocknet und im Vakuum eingedampft. Flash-Chromatographie [Kiesel gel 60 (40-63 μm), Dichlormethan/Methanol (99 :1)] ergibt das 4-(n-Butyl)-5-(2′ -cyanobiphenyl-4-ylmethyl)-

1,2-dimethyl-6-oxo-1,6-dihydro-pyrimidin, welches ohne weitere Reinigung weiterverarbeitet wird.

Beispiel 2 : Ausgehend von 4-(n-Butyl)-5-(2′-cyanobiphenyl-4-ylmethyl)-1 -ethyl-2-methyl-6-oxo-1,6-dihydro-pyrimidin, und Tributylzinnazid erhält man in analoger Weise wie in Beispiel 1 beschrieben das 4-(n-Butyl)-1-ethyl-2-methyl-6-oxo-5-[2′-(1H-tetrazol-5-yl)biphenyl-4-ylmethyl]-1,6-dihydro-pyrimidin, welches aus Acetonitril in Form von weissen Kristallen kristallisiert [Smp. : 180-182° (Zersetzung)].

Das Ausgangsmaterial kann beispielsweise wie folgt hergestellt werden :

a) Durch Alkylierung von 6-(n-Butyl)-5-(2′-cyanobiphenyl-4-ylmethyl)-4-hydroxy-2-methyl-pyrimidin mit Ethyliodid in der in Beispiel Ia) beschriebenen Weise und Flash-Chromatographie [Kieselgel 60 (40-63 µm), Hexan/Essigsäureethylester (1 :1)] erhält man das 4-(n-Butyl)-5-(2′-cyanobiphenyl-4-ylmethyl)-1-ethyl-2-methyl-6-oxo-1,6-dihydro-pyrimidin, welches direkt weiterverarbeitet wird.

Beispiel 3 : Ausgehend von 4-(n-Butyl)-5-(2′-cyanobiphenyl-4-ylmethyl)-2-methyl-6-oxo-1-(n-propyl)-1,6-dihydro-pyrimidin und Tributylzinnazid erhält man in analoger Weise wie in Beispiel 1 beschrieben das 4-(n-Butyl)-2-methyl-6-oxo- 1-(n-propyl)-5-[2′-(1H-tetrazol-5-yl)biphenyl-4-ylmethyl]-1,6-dihydro-pyrimidin, welches aus Essigsäure/Wasser in Form von weissen Kristallen kristallisiert [Smp. : 168-170° (Zersetzung)].

Das Ausgangsmaterial kann beispielsweise wie folgt hergestellt werden :

a) Durch Alkylierung von 6-(n-Butyl)-5-(2′-cyanobiphenyl-4-ylmethyl)-4-hydroxy-2-methyl-pyrimidin mit n-Propyliodid in der in Beispiel 1a) beschriebenen Weise und Flash-Chromatographie [Kieselgel 60 (40-63 µm), Hexan/Essigsäureethylester (1 :1)] erhält man das 4-(n-Butyl)-5-(2′-cyanobiphenyl-4-ylmethyl)-2-methyl-6-oxo-1-(n-propyl)-1,6-dihydro-pyrimidin, welches direkt weiterverarbeitet wird.

Beispiel 4 : Ausgehend von 4-(n-Butyl)-5-(2′-cyanobiphenyl-4-ylmethyl)-1-ethoxy-carbonylmethyl-2-methyl-6-oxo-1,6-dihydro-pyrimidin und Tributylzinnazid erhält man in analoger Weise wie in Beispiel 1 beschrieben das 4-(n-Butyl)-1 -ethoxycarbonylmethyl-2-methyl-6-oxo-5-[2′-(1H-tetrazol-5-yl)biphenyl-4-ylmethyl]-1 ,6-dihydro-pyrimidin, welches in Form von weissen Kristallen aus Diisopropylether kristallisiert [Smp. : 150-155° (Zersetzung)].

Das Ausgangsmaterial kann beispielsweise wie folgt hergestellt werden :

a) Durch Alkylierung von 6-(n-Butyl)-5-(2′-cyanobiphenyl-4-ylmethyl)-4-hydroxy-2-methyl-pyrimidin mit Bromessigsäureethylester in der in Beispiel 1a) beschriebenen Weise und Flash-Chromatographie [Kieselgel 60 (40-63 µm), Hexan/Essigsäureethylester (1 :1)] erhält man das 4-(n-Butyl)-5-(2′-cyanobiphenyl-4-ylmethyl)-1 -ethoxycarbonyl-methyl-2-methyl-6-oxo-1,6-dihydro-pyrimidin, welches direkt weiterverarbeitet wird.

Beispiel 5 : 973 mg (2 mmol) 4-(n-Butyl)-1-ethoxycarbonylmethyl-2-methyl-6-oxo-5-[2′-(1H-tetrazol-5-yl)biphenyl-4-ylmethyl]-1,6-dihydro-pyrimidin werden in 4 ml (4 mmol) 1 N-Natronlauge 12 Stunden bei Raumtemperatur gerührt. Unter Eiskühlung wird mit 1 N-Salzsäure auf pH 2 angesäuert und der Niederschlag abfiltriert. Nach Umkristallisation aus Acetonitril/Wasser erhält man das reine 4-(n-Butyl)-1-carboxymethyl-2-methyl-6-oxo-5-[2′-(1H-tetrazol-5-yl)biphenyl-4-ylmethyl]-1,6-dihydropyrimidin, das bei 137 bis 140° schmilzt.

Beispiel 6 : 870 mg (2,34 mmol) 4-(n-Butyl)-5-(2′-cyanobiphenyl-4-ylmethyl)-1,2-dimethyl-6-oxo-1,6-dihydro-pyrimidin werden in 3 ml 4 N-Kalilauge und 3 ml n-Propanol 48 Stunden unter Rückfluss gerührt. Nach dem Eindampfen des Gemisches im Vakuum wird der Rückstand in Wasser gelöst und die Lösung mit Dichlormethan extrahiert. Die wässrige Phase wird mit 4 N-Salzsäure angesäuert und der Niederschlag abfiltriert und im Vakuum über Phosphorpentoxid getrocknet. Man erhält so das 4-(n-Butyl)-5-(2′-carboxybiphenyl-4-ylmethyl)-1,2-dimethyl-6-oxo-1,6-dihydropyrimidin.

Beispiel 7 : Ausgehend von 4-(n-Butyl)-5-(2′-cyanobiphenyl-4-ylmethyl)-1-(2-ethoxyethyl)-2-methyl-6-oxo-1,6-dihydro-pyrimidin und Tributylzinnazid erhält man in der in Beispiel 1 beschriebenen Weise das 4-(n-Butyl)-1-(2-ethoxyethyl)-2-methyl-6-oxo-5-[2′-(1H-tetrazol-5-yl)biphenyl-4-ylmethyl]-1,6-dihydro-pyrimidin [Smp.: 167-179° (aus Essigsäureethylester)].

Das Ausgangsmaterial kann z. B. wie folgt hergestellt werden :

a) Durch Alkylierung von 6-(n-Butyl)-5-(2′-cyanobiphenyl-4-ylmethyl)-4-hydroxy-2-methyl-pyrimidin mit Bromethylethylether in der in Beispiel 1a) beschriebenen Weise und Flash-Chromatographie [Kieselgel 60 (40-63 µm), Hexan/Essigsäureethylester (1 :1)] erhält man 4-(n-Butyl)-5-(2′-cyanobiphenyl-4-ylmethyl)-1-(2-ethoxyethyl)-2-methyl-6-oxo-1,6-dihydro-pyrimidin, welches direkt weiterverarbeitet wird.

Beispiel 8 : Ausgehend von 4-(n-Butyl)-5-(2′-cyanobiphenyl-4-ylmethyl)-2-methyl-1-[N,N-(3-oxapent-1,5-ylen)aminocarbonylmethyl]-6-oxo-1,6-dihydro-pyrimidin und Tributylzinnazid erhält man in der in Beispiel 1 beschriebenen Weise das 4-(n-Butyl)-2-methyl-1-[N,N-(3-oxapent-1,5-ylen)aminocarbonylmethyl]-6-oxo-5-[2′-(1H-tetrazol-5-yl)biphenyl-4-ylmethyl]-1,6-dihydro-pyrimidin [Smp. : 218-221° (aus Isopropanol/Diethylether)].

Das Ausgangsmaterial kann z. B. wie folgt hergestellt werden :

a) Eine Lösung von 7,3 g (16,46 mmol) 4-(n-Butyl)-5-(2′-cyanobiphenyl-4-ylmethyl)-1-ethoxycarbonylmethyl-2-methyl-6-oxo-1,6-dihydro-pyrimidin in 150 ml Ethanol wird mit 33 ml 1 N-Natronlauge versetzt. Das Reaktionsgemisch wird 12 Stunden bei Raumtemperatur gerührt. Nach Entfernen des Ethanols im Vakuum wird die wässrige Phase mit 1 N-Salzsäure auf pH 2 angesäuert. Die ausgefallenen Kristalle werden abfiltriert.

Man erhält so das 4-(n-Butyl)-1-carboxymethyl-5-(2'-cyanobiphenyl-4-ylmethyl)-2-methyl-6-oxo-1,6-dihydro-pyrimidin [Smp. : 146-148° (aus Essigsäureethylester)].

b) Zu einer Lösung von 415 mg (1 mmol) 4-(n-Butyl)-1-carboxymethyl-5-(2'-cyanobiphenyl-4-ylmethyl)-2-methyl-6-oxo-1,6-dihydro-pyrimidin in 5 ml N,N-Dimethylformamid werden bei 0° 230 mg (1,2 mmol) N-(3-Dimethylaminopropyl)-N'-ethyl-carbodiimid-hydrochlorid, 203 mg (1,5 mmol) Hydroxybenzotriazol und 0,175 ml (2 mmol) Morpholin zugegeben. Das Reaktionsgemisch wird 12 Stunden bei Raumtemperatur gerührt und anschliessend im Vakuum eingedampft. Der Rückstand wird in Essigsäureethylester gelöst und die Lösung mit 0,1 N-Salzsäure, gesättigter $NaHCO_3$-Lösung und gesättigter NaCl-Lösung gewaschen, getrocknet ($Na_2SO_4$) und im Vakuum eingedampft. Man erhält so 4-(n-Butyl)-5-(2'-cyanobiphenyl-4-ylmethyl)-2-methyl-1-[N,N-(3-oxapent-1,5-ylen)aminocarbonylmethyl]-6-oxo-1,6-dihydro-pyrimidin, welches direkt weiterverarbeitet wird.

Beispiel 9 : In analoger Weise wie in einem der vorstehenden Beispiele beschrieben kann man auch herstellen :

1. das 1,4-Di-(n-butyl)-2-methyl-6-oxo-5-[2'-(1H-tetrazol-5-yl)biphenyl-4-ylmethyl]-1,6-dihydro-pyrimidin, Smp. : 140-142° ;
2. das 1-Benzyl-4-(n-butyl)-2-methyl-6-oxo-5-[2'-(1H-tetrazol-5-yl)biphenyl-4-ylmethyl]-1,6-dihydro-pyrimidin, amorph ;
3. das 4-(n-Butyl)-2-methyl-6-oxo-1-(2-phenylethyl)-5-[2'-(1H-tetrazol-5-yl)biphenyl-4-ylmethyl]-1,6-dihydro-pyrimidin, amorph ;
4. das 4-(n-Butyl)-1-(2-hydroxyethyl)-2-methyl-6-oxo-5-[2'-(1H-tetrazol-5-yl)biphenyl-4-ylmethyl]-1,6-dihydro-pyrimidin, Smp. : 188-191° ;
5. das 4-(n-Butyl)-1-[2-(2-methoxyethoxy)ethyl]-2-methyl-6-oxo-5-[2'-(1H-tetrazol-5-yl)-biphenyl-4-ylmethyl]-1,6-dihydro-pyrimidin, Smp. : 132-134° ;
6. das 4-(n-Butyl)-2-ethyl-1-methyl-6-oxo-5-[2'-(1H-tetrazol-5-yl)biphenyl-4-ylmethyl]-1,6-dihydro-pyrimidin ;
7. das 4-(n-Butyl)-1-methyl-6-oxo-2-(n-propyl)-5-[2'-(1H-tetrazol-5-yl)biphenyl-4-ylmethyl]-1,6-dihydro-pyrimidin, Smp. : 161-163° ;
8. das 2,4-Di-(n-butyl)-1-methyl-6-oxo-5-[2'-(1H-tetrazol-5-yl)biphenyl-4-ylmethyl]-1,6-dihydro-pyrimidin, Smp. : 157-159° ;
9. das 4-(n-Butyl)-2-isopropyl-1-methyl-6-oxo-5-[2'-(1H-tetrazol-5-yl)biphenyl-4-ylmethyl]-1,6-dihydro-pyrimidin ;
10. das 4-(n-Butyl)-1,2-diethyl-6-oxo-5-[2'-(1H-tetrazol-5-yl)biphenyl-4-ylmethyl]-1,6-dihydro-pyrimidin ;
11. das 4-(n-Butyl)-1-ethyl-6-oxo-2-(n-propyl)-5-[2'-(1H-tetrazol-5-yl)biphenyl-4-ylmethyl]-1,6-dihydro-pyrimidin ;
12. das 2,4-Di-(n-butyl)-1-ethyl-6-oxo-5-[2'-(1H-tetrazol-5-yl)biphenyl-4-ylmethyl]-1,6-dihydro-pyrimidin, Smp. : 113-116° ;
13. das 4-(n-Butyl)-1-ethyl-2-isopropyl-6-oxo-5-[2'-(1H-tetrazol-5-yl)biphenyl-4-ylmethyl]-1,6-dihydro-pyrimidin ;
14. das 4-(n-Butyl)-2-ethyl-6-oxo-1-(n-propyl)-5-[2'-(1H-tetrazol-5-yl)-biphenyl-4-ylmethyl]-1,6-dihydro-pyrimidin ;
15. das 4-(n-Butyl)-1,2-di-(n-propyl)-6-oxo-5-[2'-(1H-tetrazol-5-yl)biphenyl-4-ylmethyl]-1,6-dihydro-pyrimidin ;
16. das 2,4-Di-(n-butyl)-6-oxo-1-(n-propyl)-5-[2'-(1H-tetrazol-5-yl)biphenyl-4-ylmethyl]-1,6-dihydro-pyrimidin ;
17. das 4-(n-Butyl)-2-isopropyl-6-oxo-1-(n-propyl)-5-[2'-(1H-tetrazol-5-yl)biphenyl-4-ylmethyl]-1,6-dihydro-pyrimidin ;
18. das 1,4-Di-(n-butyl)-2-ethyl-6-oxo-5-[2'-(1H-tetrazol-5-yl)biphenyl-4-ylmethyl]-1,6-dihydro-pyrimidin ;
19. das 1,4-Di-(n-butyl)-6-oxo-2-(n-propyl)-5-[2'-(1H-tetrazol-5-yl)biphenyl-4-ylmethyl]-1,6-dihydro-pyrimidin ;
20. das 6-Oxo-5-[2'-(1H-tetrazol-5-yl)biphenyl-4-ylmethyl]-1,2,4-tri-(n-butyl)-1,6-dihydro-pyrimidin ;
21. das 1,4-Di-(n-butyl)-2-isopropyl-6-oxo-5-[2'-(1H-tetrazol-5-yl)biphenyl-4-ylmethyl]-1,6-dihydro-pyrimidin ;
22. das 1,2-Dimethyl-6-oxo-4-(n-propyl)-5-(2'-carboxybiphenyl-4-ylmethyl)-1,6-dihydro-pyrimidin ;
23. das 1-Benzylaminocarbonylmethyl-4-(n-butyl)-2-methyl-6-oxo-5-[2'-(1H-tetrazol-5-yl)biphenyl-4-ylmethyl]-1,6-dihydro-pyrimidin, Smp : 215-221° ;
24. das 4-(n-Butyl)-2-methyl-6-oxo-1-(2-phenylethylaminocarbonylmethyl)-5-[2'-(1H-tetrazol-5-yl)biphenyl-4-ylmethyl]-1,6-dihydro-pyrimidin, Smp : 121-126° ;
25. das 4-(n-Butyl)-1-(n-butylaminocarbonylmethyl)-2-methyl-6-oxo-5-[2'-(1H-tetrazol-5-yl)biphenyl-4-ylmethyl]-1,6-dihydro-pyrimidin, Smp : 239-241° ;

26. das 4-(n-Butyl)-2-methyl-1-methylaminocarbonylmethyl-6-oxo-5-[2'-(1H-tetrazol-5-yl)biphenyl-4-ylmethyl]-1,6-dihydro-pyrimidin, Smp : 245-247° ;

27. das 4-(n-Butyl)-1-dimethylaminocarbonylmethyl-2-methyl-6-oxo-5-[2'-(1H-tetrazol-5-yl)biphenyl-4-ylmethyl]-1,6-dihydro-pyrimidin, Smp : 219-222° ;

28. das 4-(n-Butyl)-6-oxo-1,2-tetramethylen-5-[2'-(1H-tetrazol-5-yl)biphenyl-4-ylmethyl]-1,6-dihydro-pyrimidin, Smp : 205-206° und

29. das 4-(n-Butyl)-6-oxo-5-[2'-(1H-tetrazol-5-yl)biphenyl-4-ylmethyl]-1,2-trimethylen-1,6-dihydro-pyrimidin, Smp : 222-224°.

Beispiel 10 : Tabletten, enthaltend je 50 mg Wirkstoff, z.B. 4-(n-Butyl)-1,2-dimethyl-6-oxo-5-[2'-(1H-tetrazol-5-yl)biphenyl-4-ylmethyl]-1,6-dihydro-pyrimidin, können wie folgt hergestellt werden :

Zusammensetzung (für 10000 Tabletten) :

| | |
|---|---|
| Wirkstoff | 500,0 g |
| Lactose | 500,0 g |
| Kartoffelstärke | 352,0 g |
| Gelatine | 8,0 g |
| Talk | 60,0 g |
| Magnesiumstearat | 10,0 g |
| Siliciumdioxid (hochdispers) | 20,0 g |
| Ethanol | q.s. |

Der Wirkstoff wird mit der Lactose und 292 g Kartoffelstärke vermischt, die Mischung mit einer alkoholischen Lösung der Gelatine befeuchtet und durch ein Sieb granuliert. Nach dem Trocknen mischt man den Rest der Kartoffelstärke, den Talk, das Magnesiumstearat und das hochdisperse Siliciumdioxid zu und presst das Gemisch zu Tabletten von je 145,0 mg Gewicht und 50,0 mg Wirkstoffgehalt, die gewünschtenfalls mit Teilkerben zur feineren Anpassung der Dosierung versehen sein können.

Beispiel 11 : Lacktabletten, enthaltend je 100 mg Wirkstoff, z.B. 4-(n-Butyl)-1,2-dimethyl-6-oxo-5-[2'-(1H-tetrazol-5-yl)biphenyl-4-ylmethyl]-1,6-dihydro-pyrimidin, können wie folgt hergestellt werden :

Zusammensetzung (für 1000 Tabletten) :

| | |
|---|---|
| Wirkstoff | 100,00 g |
| Lactose | 100,00 g |
| Maisstärke | 70,00 g |
| Talk | 8,50 g |
| Calciumstearat | 1,50 g |
| Hydroxypropylmethylcellulose | 2,36 g |
| Schellack | 0,64 g |
| Wasser | q.s. |
| Dichlormethan | q. s. |

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat werden zugegeben und mit dem Granulat vermischt. Das Gemisch wird zu Tabletten (Gewicht : 280 mg) verpresst und diese mit einer Lösung der Hydroxypropylmethylcellulose und des Schellacks in Dichlormethan lackiert (Endgewicht der Lacktablette : 283 mg).

Beispiel 12 : In analoger Weise wie in den Beispielen 10 und 11 beschrieben können auch Tabletten und Lacktabletten, enthaltend eine andere Verbindung der Formel I oder ein pharmazeutisch verwendbares Salz einer Verbindung der Formel I, z.B. gemäss einem der Beispiele 1 bis 9, hergestellt werden.

## Patentansprüche

1. Eine Verbindung der Formel

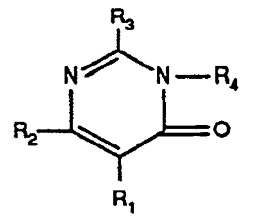

(I),

worin einer der Reste $R_1$ und $R_2$ einen gegebenenfalls durch Halogen oder Hydroxy substituierten aliphatischen Kohlenwasserstoffrest oder einen cycloaliphatischen oder araliphatischen Kohlenwasserstoffrest bedeutet, der andere der Reste $R_1$ und $R_2$ für die Gruppe der Formel

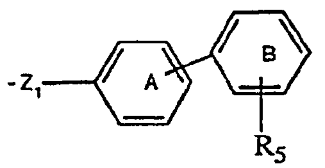

(Ia)

steht, in der $Z_1$ für Alkylen, O, $S(O)_m$ oder N(R) steht, $R_5$ Carboxy, Halogenalkansulfonylamino, $SO_3H$, $PO_2H_2$, $PO_3H_2$ oder 5-Tetrazolyl bedeutet und die Ringe A und B unabhängig voneinander gegebenenfalls substituiert sind durch einen aliphatischen Kohlenwasserstoffrest, der gegebenenfalls durch O unterbrochen und gegebenenfalls durch Hydroxy oder Halogen substituiert ist, gegebenenfalls durch einen aliphatischen Alkohol verethertes Hydroxy, Halogen, gegebenenfalls verestertes oder amidiertes Carboxy oder 5-Tetrazolyl, und entweder $R_3$ Halogen, Acyl, einen aromatischen Kohlenwasserstoffrest, gegebenenfalls verestertes oder amidiertes Carboxy, Cyano, $SO_3H$, $PO_3H_2$, $PO_2H_2$, 5-Tetrazolyl, gegebenenfalls substituiertes Sulfamoyl oder Acylamino bedeutet oder für $-Z_2-R'$ steht, worin $Z_2$ für eine Bindung oder für O, $S(O)_m$ oder N(R) steht und R' Wasserstoff oder einen aliphatischen Kohlenwasserstoffrest bedeutet, der gegebenenfalls durch O oder $S(O)_m$ unterbrochen ist und gegebenenfalls durch Halogen, Hydroxy, gegebenenfalls substituiertes Amino oder gegebenenfalls verestertes oder amidiertes Carboxy substituiert ist, und $R_4$ einen gegebenenfalls durch O oder $S(O)_m$ unterbrochenen aliphatischen Kohlenwasserstoffrest bedeutet, der gegebenenfalls durch gegebenenfalls verestertes oder amidiertes Carboxy, gegebenenfalls mit einem aromatischen Alkohol verethertes Hydroxy, gegebenenfalls substituiertes Amino, $S(O)_m$-R oder einen aromatischen Kohlenwasserstoffrest substituiert ist, oder $R_3$ und $R_4$ gemeinsam für Alkylen stehen, wobei R jeweils Wasserstoff oder einen aliphatischen Kohlenwasserstoffrest bedeutet und m jeweils für 0, 1 oder 2 steht, in freier Form oder in Salzform.

2. Eine Verbindung gemäss Anspruch 1 der Formel I, worin einer der Reste $R_1$ und $R_2$ jeweils gegebenenfalls durch Halogen oder Hydroxy substituiertes Niederalkyl, Niederalkenyl oder Niederalkinyl, jeweils 3- bis 7-gliedriges Cycloalkyl oder Cycloalkenyl, Phenylniederalkyl, Phenylniederalkenyl oder Phenylniederalkinyl bedeutet, der andere der Reste $R_1$ und $R_2$ für die Gruppe Ia steht, in der $Z_1$ für Methylen, Niederalkylen, O, $S(O)_m$ oder N(R) steht, $R_5$ Carboxy, Halogenniederalkansulfonylamino, $SO_3H$, $PO_2H_2$, $PO_3H_2$ oder 5-Tetrazolyl bedeutet und die Ringe A und B unabhängig voneinander gegebenenfalls substituiert sind durch Halogen, durch jeweils gegebenenfalls durch Halogen oder Hydroxy substituiertes Niederalkyl, Nedeialkenyl, Niederalkinyl, Niederalkoxyniederalkyl, Niederalkoxyniederalkenyl, Niederalkoxyniederalkinyl, Niederalkenyloxyniederalkyl, Niederalkenyloxyniederalkenyl, Niederalkenyloxyniederalkinyl oder Niederalkoxyniederalkoxyniederalkyl, durch Hydroxy, durch Niederalkoxy, durch Niederalkenyloxy, durch Carboxy, welches gegebenenfalls durch einen Alkohol verestert ist, der sich von Niederalkyl, Niederalkenyl, Niederalkinyl, Niederalkoxyniederalkyl, Niederalkoxyniederalkenyl oder Niederalkoxyniederalkinyl ableitet, durch Carbamoyl, in welchem die Aminogruppe gegebenenfalls durch Niederalkyl, Niederalkenyl, Niederalkinyl, Phenylniederalkyl, Phenylniederalkenyl oder Phenylniederalkinyl unabhängig voneinander mono- oder di-substituiert oder durch Niederalkylen oder Niederalkylenoxyniederalkylen disubstituiert ist, oder durch 5-Tetrazolyl, und entweder $R_3$ Halogen, Niederalkanoyl, gegebenenfalls substituiertes Phenyl, Carboxy, welches gegebenenfalls durch einen Alkohol verestert ist, der sich von Niederalkyl, Niederalkenyl, Niederalkinyl, Niederalkoxyniederalkyl, Niederalkoxyniederalkenyl oder Niederalkoxyniederalkinyl ableitet, Carbamoyl, in welchem die Aminogruppe gegebenenfalls durch Niederalkyl, Niederalkenyl, Niederalkinyl, Phenylniederalkyl, Phenylniederalkenyl oder Phenylniederalkinyl unabhängig voneinander

mono- oder di-substituiert ist oder durch Niederalkylen oder Niederalkylenoxyniederalkylen disubstituiert ist, Cyano, $SO_3H$, $PO_2H_2$, $PO_3H_2$, 5-Tetrazolyl, Sulfamoyl, in dem die Aminogruppe gegebenenfalls durch Niederalkyl mono- oder disubstituiert ist, Niederalkansulfonylamino, Halogenniederalkansulfonylamino, Niederalkanoylamino, gegebenenfalls substituiertes Benzoylamino oder gegebenenfalls substituiertes Benzolsulfonylamino bedeutet oder für $-Z_2-R'$ steht, worin $Z_2$ für eine Bindung oder für O, $S(O)_m$ oder N(R) steht und R' Wasserstoff oder jeweils gegebenenfalls durch Hydroxy, Halogen, Amino, Niederalkylenamino, Niederalkylenoxyniederalkylenamino, Niederalkylamino, Niederalkenylamino, Niederalkinylamino, Phenylniederalkylamino, Phenylniederalkenylamino, Phenylniederalkinylamino, Diniederalkylamino, N-Niederalkyl-N-phenylniederalkyl-amino, Di(phenylniederalkyl)amino, Carboxy, welches gegebenenfalls durch einen Alkohol verestert ist, der sich von Niederalkyl, Niederalkenyl, Niederalkinyl, Niederalkoxyniederalkyl, Niederalkoxyniederalkenyl oder Niederalkoxyniederalkinyl ableitet, oder Carbamoyl, in welchem die Aminogruppe gegebenenfalls durch Niederalkyl, Niederalkenyl, Niederalkinyl, Phenylniederalkyl, Phenylniederalkenyl oder Phenylniederalkinyl unabhängig voneinander mono- oder di-substituiert oder durch Niederalkylen oder Niederalkylenoxyniederalkylen disubstituiert ist, substituiertes Niederalkyl, Niederalkenyl, Niederalkinyl, Niederalkoxyniederalkyl, Niederalkoxyniederalkenyl, Niederalkoxyniederalkinyl, Niederalkenyloxyniederalkyl, Niederalkenyloxyniederalkenyl, Niederalkenyloxyniederalkinyl, Niederalkoxyniederalkoxyniederalkyl, Niederalkylthio-niederalkyl, -niederalkenyl oder -niederalkinyl, Niederalkan-sulfinylniederalkyl oder -sulfonylniederalkyl, Niederalkenyl-thioniederalkyl, -sulfinylniederalkyl oder -sulfonylniederalkyl oder Niederalkinyl-thioniederalkyl, -sulfinylniederalkyl oder -sulfonylniederalkyl bedeutet, und $R_4$ jeweils gegebenenfalls durch Hydroxy, $S(O)_m$-R, jeweils gegebenenfalls substituiertes Phenyl, Phenoxy oder Naphthyloxy, Amino, Niederalkylenamino, Niederalkylenoxyniederalkylenamino, Niederalkylamino, Niederalkenylamino, Niederalkinylamino, Phenylniederalkylamino, Phenylniederalkenylamino, Phenylniederalkinylamino, Diniederalkylamino, N-Niederalkyl-N-phenylniederalkyl-amino, Di(phenylniederalkyl)amino, Carboxy, welches gegebenenfalls durch einen Alkohol verestert ist, der sich von Niederalkyl, Niederalkenyl, Niederalkinyl, Niederalkoxyniederalkyl, Niederalkoxyniederalkenyl oder Niederalkoxyniederalkinyl ableitet, oder Carbamoyl, in welchem die Aminogruppe gegebenenfalls durch Niederalkyl, Niederalkenyl, Niederalkinyl, Phenylniederalkyl, Phenylniederalkenyl oder Phenylniederalkinyl unabhängig voneinander mono- oder di-substituiert oder durch Niederalkylen oder Niederalkylenoxyniederalkylen disubstituiert ist, substituiertes Niederalkyl, Niederalkenyl, Niederalkinyl, Niederalkoxyniederalkyl, Niederalkoxyniederalkenyl, Niederalkoxyniederalkinyl, Niederalkenyloxyniederalkyl, Niederalkenyloxyniederalkenyl, Niederalkenyloxyniederalkinyl, Niederalkoxyniederalkoxyniederakyl, Niederalkylthio-niederalkyl, -niederalkenyl oder -niederalkinyl, Niederalkan-sulfinylniederalkyl oder -sulfonylniederalkyl, Niederalkenyl-thioniederalkyl, -sulfinylniederalkyl oder - sulfonylniederalkyl oder Niederalkinyl-thioniederalkyl, -sulfinylniederalkyl oder -sulfonylniederalkyl bedeutet, oder $R_3$ und $R_4$ gemeinsam für Methylen oder Niederalkylen stehen, wobei R jeweils Wasserstoff, Niederalkyl, Niederalkenyl oder Niederalkinyl bedeutet, m jeweils für 0, 1 oder 2 steht und aromatische Reste jeweils gegebenenfalls durch Niederalkyl, Niederalkoxy, Halogen, Trifluormethyl und/oder Hydroxy substituiert sind, in freier Form oder in Salzform.

3. Eine Verbindung gemäss Anspruch 1 der Formel I, worin $R_1$ für die Gruppe Ia steht, in der $Z_1$ für Methylen, Niederalkylen, O, $S(O)_m$ oder N(R) steht, $R_5$ Carboxy oder 5-Tetrazolyl bedeutet und die Ringe A und B unabhängig voneinander gegebenenfalls durch Halogen, Niederalkyl, Niederalkoxy, Carboxy, Niederalkoxycarbonyl oder 5-Tetrazolyl substituiert sind, $R_2$ jeweils gegebenenfalls durch Hydroxy oder Halogen substituiertes Niederalkyl oder Niederalkenyl bedeutet und entweder $R_3$ Halogen, Carboxy, welches gegebenenfalls durch einen Alkohol verestert ist, der sich von Niederalkyl oder Niederalkoxyniederalkyl ableitet, Carbamoyl, Cyano, $PO_3H_2$, 5-Tetrazolyl, Niederalkansulfamoyl, Niederalkanoylamino oder Niederalkansulfonylamino bedeutet oder für $-Z_2-R'$ steht, worin $Z_2$ für eine Bindung oder für O, $S(O)_m$ oder N(R) steht und R' Wasserstoff oder jeweils gegebenenfalls durch Carboxy, Niederalkoxycarbonyl, Niederalkoxyniederalkoxycarbonyl oder Hydroxy substituiertes Niederalkyl oder Niederalkoxyniederalkyl bedeutet, und $R_4$ Niederalkyl, Niederalkenyl, Niederalkoxyniederalkyl, Carboxyniederalkyl, Niederalkoxycarbonylniederalkyl, Niederalkoxyniederalkoxycarbonylniederalkyl, Carbamoyl-, Niederalkylcarbamoyl- oder Diniederalkylcarbamoyl-niederalkyl, Hydroxyniederalkyl, gegebenenfalls substituiertes Phenoxyniederalkyl, Amino-, Niederalkylamino- oder Diniederalkylamino-niederalkyl oder gegebenenfalls substituiertes Phenylniederalkyl bedeutet, oder $R_3$ und $R_4$ gemeinsam für Methylen oder Niederalkylen stehen, wobei R jeweils Wasserstoff oder Niederalkyl bedeutet, m jeweils 0, 1 oder 2 bedeutet und aromatische Reste jeweils gegebenenfalls durch Niederalkyl, Niederalkoxy, Halogen, Trifluormethyl und/oder Hydroxy substituiert sind, in freier Form oder in Salzform.

4. Eine Verbindung gemäss Anspruch 1 der Formel I, worin $R_1$ für die Gruppe Ia steht, in der $Z_1$ für Methylen, Niederalkylen, O oder $S(O)_m$ steht, $R_5$ Carboxy oder 5-Tetrazolyl bedeutet und die Ringe A und B unabhängig voneinander gegebenenfalls durch Niederalkyl, Halogen oder Niederalkoxy substituiert sind, $R_2$ jeweils gegebenenfalls durch Hydroxy oder Halogen substituiertes Niederalkyl oder Niederalkenyl bedeutet und entweder $R_3$ Halogen, Carboxy, Niederalkoxycarbonyl, $PO_3H_2$, 5-Tetrazolyl, Niederalkanoylamino, Niederalkansulfonylamino, Wasserstoff, Niederalkyl, Niederalkoxyniederalkyl, Carboxyniederalkyl, Niederalkoxycarbonylniederalkyl, Hydroxyniederalkyl, Hydroxyniederalkoxyniederalkyl, Hydroxy, Niederalkoxy, Niederalkoxyniederalkoxy, Carboxyniederalkoxy, Niederalkoxycarbonylniederalkoxy, Hydroxyniederalkoxy, Mercapto, Niederalkylthio, Niederalkansulfinyl, Niederalkansulfonyl, Amino, Niederalkylamino oder Diniederalkylamino bedeutet und $R_4$ Niederalkyl, Hydroxyniederalkyl, Carboxyniederalkyl oder Niederalkoxycarbonylniederalkyl bedeutet oder $R_3$ und $R_4$ gemeinsam für Methylen oder Niederalkylen stehen, wobei m für 0, 1 oder 2 steht und mit "nieder" bezeichnete Teilstrukturen jeweils bis und mit 7 C-Atome umfassen, in freier Form oder in Salzform.

5. Eine Verbindung gemäss Anspruch 1 der Formel I, worin $R_1$ für die Gruppe Ia steht, in der $Z_1$ für Methylen oder Niederalkylen steht, $R_5$ Carboxy oder 5-Tetrazolyl bedeutet und die Ringe A und B unabhängig voneinander gegebenenfalls durch Niederalkyl, Halogen, mit einer Atomnummer bis und mit 35 oder Niederalkoxy substituiert sind, $R_2$ Niederalkyl bedeutet, $R_3$ Halogen, mit einer Atomnummer bis und mit 35, Wasserstoff, Niederalkyl, Niederalkoxyniederalkyl, Carboxyniederalkyl, Niederalkoxycarbonylniederalkyl, Hydroxyniederalkyl, Hydroxyniederalkoxyniederalkyl, Hydroxy, Niederalkoxy, Niederalkoxyniederalkoxy, Carboxyniederalkoxy, Niederalkoxycarbonylniederalkoxy, Hydroxyniederalkoxy oder Niederalkoxyniederalkoxy bedeutet und $R_4$ Niederalkyl, Hydroxyniederalkyl, Carboxyniederalkyl oder Niederalkoxycarbonylniederalkyl bedeutet, wobei mit "nieder" bezeichnete Teilstrukturen jeweils bis und mit 4 C-Atome umfassen, in freier Form oder in Salzform.

6. Eine Verbindung gemäss einem der Ansprüche 1 bis 5 der Formel I, worin $R_1$ für die Gruppe der Formel

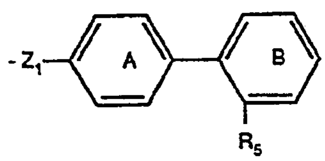

(Ib)

steht, in freier Form oder in Salzform.

7. Eine Verbindung gemäss Anspruch 1 der Formel I, worin $R_1$ für die Gruppe Ib steht, in der $Z_1$ Methylen bedeutet, $R_5$ Carboxy oder 5-Tetrazolyl bedeutet und die Ringe A und B unsubstituiert sind, $R_2$ $C_3$-$C_7$-Alkyl bedeutet, $R_3$ Wasserstoff oder $C_1$-$C_4$-Alkyl bedeutet und $R_4$ $C_1$-$C_4$-Alkyl, Carboxy-$C_1$-$C_4$-alkyl, $C_1$-$C_4$-Alkoxycarbonyl-$C_1$-$C_4$-alkyl oder Hydroxy-$C_1$-$C_4$-alkyl bedeutet, in freier Form oder in Salzform.

8. Eine Verbindung gemäss Anspruch 1 der Formel I, worin $R_1$ für die Gruppe Ib steht, in der $Z_1$ Methylen bedeutet, $R_5$ Carboxy oder Tetrazolyl bedeutet und die Ringe A und B unsubstituiert sind, $R_2$ $C_3$-$C_5$-Alkyl bedeutet, $R_3$ Wasserstoff oder $C_1$-$C_4$-Alkyl bedeutet und $R_4$ $C_1$-$C_4$-Alkyl bedeutet, in freier Form oder in Salzform.

9. 4-(n-Butyl)-1,2-dimethyl-6-oxo-5-[2'-(1H-tetrazol-5-yl)biphenyl-4-ylmethyl]-1,6-dihydro-pyrimidin oder ein Salz davon.

10. 4-(n-Butyl)-1-ethyl-2-methyl-6-oxo-5-[2'-(1H-tetrazol-5-yl)biphenyl-4-ylmethyl]-1,6-dihydro-pyrimidin oder ein Salz davon.

11. 4-(n-Butyl)-2-methyl-6-oxo-1-(n-propyl)-5-[2'-(1H-tetrazol-5-yl)biphenyl-4-ylmethyl]-1,6-dihydro-pyrimidin oder ein Salz davon.

12. 4-(n-Butyl)-1-ethoxycarbonylmethyl-2-methyl-6-oxo-5-[2'-(1H-tetrazol-5-yl)biphenyl-4-ylmethyl]-1,6-dihy dro-pyrimidin oder ein Salz davon.

13. 4-(n-Butyl)-1-(2-ethoxyethyl)-2-methyl-6-oxo-5-[2′-(1H-tetrazol-5-yl)biphenyl-4-ylmethyl]-1,6-dihydro-pyrimidin,
1,4-Di-(n-butyl)-2-methyl-6-oxo-5-[2′-(1H-tetrazol-5-yl)biphenyl-4-ylmethyl]-1,6-dihydro-pyrimidin,
1-Benzyl-4-(n-butyl)-2-methyl-6-oxo-5-[2′-(1H-tetrazol-5-yl)biphenyl-4-ylmethyl]-1,6-dihydro-pyrimidin,
4-(n-Butyl)-2-methyl-6-oxo-1-(2-phenylethyl)-5-[2′-(1H-tetrazol-5-yl)biphenyl-4-ylmethyl]-1,6-dihydro-pyrimidin,
4-(n-Butyl)-1-(2-hydroxyethyl)-2-methyl-6-oxo-5-[2′-(1H-tetrazol-5-yl)biphenyl-4-ylmethyl]-1,6-dihydro-pyrimidin oder
4-(n-Butyl)-1-methyl-6-oxo-2-(n-propyl)-5-[2′-(1H-tetrazol-5-yl)biphenyl-4-ylmethyl]-1,6-dihydro-pyrimidin oder jeweils ein Salz davon.

14. 2,4-Di-(n-butyl)-1-methyl-6-oxo-5-[2′-(1H-tetrazol-5-yl)biphenyl-4-ylmethyl]-1,6-dihydro-pyrimidin,
4-(n-Butyl)-2-isopropyl-1-methyl-6-oxo-5-[2′-(1H-tetrazol-5-yl)biphenyl-4-ylmethyl]-1,6-dihydro-pyrimidin,
4-(n-Butyl)-1,2-diethyl-6-oxo-5-[2′-(1H-tetrazol-5-yl)biphenyl-4-ylmethyl]-1,6-dihydro-pyrimidin,
4-(n-Butyl)-1-ethyl-6-oxo-2-(n-propyl)-5-[2′-(1H-tetrazol-5-yl)biphenyl-4-ylmethyl]-1,6-dihydro-pyrimidin,
2,4-Di-(n-butyl)-1-ethyl-6-oxo-5-[2′-(1H-tetrazol-5-yl)biphenyl-4-ylmethyl]-1,6-dihydro-pyrimidin,
4-(n-Butyl)-1-ethyl-2-isopropyl-6-oxo-5-[2′-(1H-tetrazol-5-yl)biphenyl-4-ylmethyl]-1,6-dihydro-pyrimidin,
4-(n-Butyl)-2-ethyl-6-oxo-1-(n-propyl)-5-[2′-(1H-tetrazol-5-yl)biphenyl-4-ylmethyl]-1,6-dihydro-pyrimidin,
4-(n-Butyl)-1,2-di-(n-propyl)-6-oxo-5-[2′-(1H-tetrazol-5-yl)biphenyl-4-ylmethyl]-1,6-dihydro-pyrimidin,
2,4-Di-(n-butyl)-6-oxo-1-(n-propyl)-5-[2′-(1H-tetrazol-5-yl)biphenyl-4-ylmethyl]-1,6-dihydro-pyrimidin,
4-(n-Butyl)-2-isopropyl-6-oxo-1-(n-propyl)-5-[2′-(1H-tetrazol-5-yl)biphenyl-4-ylmethyl]-1,6-dihydro-pyrimidin,
1,4-Di-(n-butyl)-2-ethyl-6-oxo-5-[2′-(1H-tetrazol-5-yl)biphenyl-4-ylmethyl]-1,6-dihydro-pyrimidin,
1,4-Di-(n-butyl)-6-oxo-2-(n-propyl)-5-[2′-(1H-tetrazol-5-yl)biphenyl-4-ylmethyl]-1,6-dihydro-pyrimidin,
6-Oxo-5-[2′-(1H-tetrazol-5-yl)biphenyl-4-ylmethyl]-1,2,4-tri-(n-butyl)-1,6-dihydro-pyrimidin,
1,4-Di-(n-butyl)-2-isopropyl-6-oxo-5-[2′-(1H-tetrazol-5-yl)biphenyl-4-ylmethyl]-1,6-dihydro-pyrimidin,
1,2-Dimethyl-6-oxo-4-(n-propyl)-5-(2′-carboxybiphenyl-4-ylmethyl)-1,6-dihydro-pyrimidin,
4-(n-Butyl)-1-carboxymethyl-2-methyl-6-oxo-5-[2′-(1H-tetrazol-5-yl)biphenyl-4-ylmethyl]-1,6-dihydro-pyrimidin,
4-(n-Butyl)-5-(2′-carboxybiphenyl-4-ylmethyl)-1,2-dimethyl-6-oxo-1,6-dihydro-pyrimidin oder
4-(n-Butyl)-2-ethyl-1-methyl-6-oxo-5-[2′-(1H-tetrazol-5-yl)biphenyl-4-ylmethyl]-1,6-dihydro-pyrimidin oder jeweils ein Salz davon.

15. 4-(n-Butyl)-2-methyl-1-[N,N-(3-oxapent-1,5-ylen)aminocarbonylmethyl]-6-oxo-5-[2′-(1H-tetrazol-5-yl)biphenyl-4-ylmethyl]-1,6-dihydro-pyrimidin,
4-(n-Butyl)-1-[2-(2-methoxyethoxy)ethyl]-2-methyl-6-oxo-5-[2′-(1H-tetrazol-5-yl)biphenyl-4-ylmethyl]-1,6-dihydro-pyrimidin,
1-Benzylaminocarbonylmethyl-4-(n-butyl)-2-methyl-6-oxo-5-[2′-(1H-tetrazol-5-yl)biphenyl-4-ylmethyl]-1,6-dihydro-pyrimidin,
4-(n-Butyl)-2-methyl-6-oxo-1-(2-phenylethylaminocarbonylmethyl)-5-[2′-(1H-tetrazol-5-yl)biphenyl-4-ylmethyl]-1,6-dihydro-pyrimidin,
4-(n-Butyl)-1-(n-butylaminocarbonylmethyl)-2-methyl-6-oxo-5-[2′-(1H-tetrazol-5-yl)biphenyl-4-ylmethyl]-1,6-dihydro-pyrimidin,
4-(n-Butyl)-2-methyl-1-methylaminocarbonylmethyl-6-oxo-5-[2′-(1H-tetrazol-5-yl)biphenyl-4-ylmethyl]-1,6-dihydro-pyrimidin,
4-(n-Butyl)-1-dimethylaminocarbonylmethyl-2-methyl-6-oxo-5-[2′-(1H-tetrazol-5-yl)biphenyl-4-ylmethyl]-1,6-dihydro-pyrimidin,
4-(n-Butyl)-6-oxo-1,2-tetramethylen-5-[2′-(1H-tetrazol-5-yl)biphenyl-4-ylmethyl]-1,6-dihydro-pyrimidin oder
4-(n-Butyl)-6-oxo-5-[2′-(1H-tetrazol-5-yl)biphenyl-4-ylmethyl]-1,2-trimethylen-1,6-dihydro-pyrimidin oder jeweils ein Salz davon.

16. Eine Verbindung gemäss einem der Ansprüche 1 bis 15, in freier Form oder in Form eines pharmazeutisch verwendbaren Salzes, zur Anwendung in einem Verfahren zur therapeutischen Behandlung des menschlichen oder tierischen Körpers.

17. Eine Verbindung gemäss einem der Ansprüche 5 und 9 bis 14, in freier Form oder in Form eines pharmazeutisch verwendbaren Salzes, zur Anwendung in einem Verfahren zur therapeutischen Behandlung des

menschlichen oder berischen Körpers.

18. Eine Verbindung gemäss einem der Ansprüche 1 bis 17, in freier Form oder in Form eines pharmazeutisch verwendbaren Salzes, zur Anwendung als Antihypertensivum.

19. Eine Verbindung gemäss einem der Ansprüche 5, 9 bis 14 und 17, in freier Form oder in Form eines pharmazeutisch verwendbaren Salzes, zur Anwendung als Antihypertensivum.

20. Ein pharmazeutisches Präparat, als Wirkstoff enthaltend eine Verbindung gemäss einem der Ansprüche 1 bis 19, in freier Form oder in Form eines pharmazeutisch verwendbaren Salzes, gegebenenfalls neben üblichen pharmazeutischen Hilfsstoffen.

21. Ein pharmazeutisches Präparat, als Wirkstoff enthaltend eine Verbindung gemäss einem der Ansprüche 5, 9 bis 14, 17 und 19, in freier Form oder in Form eines pharmazeutisch verwendbaren Salzes, gegebenenfalls neben üblichen pharmazeutischen Hilfsstoffen.

22. Ein antihypertensiv wirksames pharmazeutisches Präparat gemäss Anspruch 20 oder 21, dadurch gekennzeichnet, dass man einen antihypertensiv wirksamen Wirkstoff wählt.

23. Ein antihypertensiv wirksames pharmazeutisches Präparat gemäss Anspruch 21, dadurch gekennzeichnet, dass man einen antihypertensiv wirksamen Wirkstoff wählt.

24. Verfahren zur Herstellung einer Verbindung der Formel

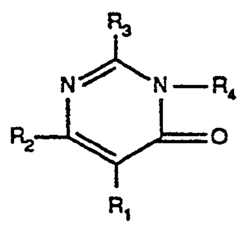

(I),

worin einer der Reste $R_1$ und $R_2$ einen gegebenenfalls durch Halogen oder Hydroxy substituierten aliphatischen Kohlenwasserstoffrest oder einen cycloaliphatischen oder araliphatischen Kohlenwasserstoffrest bedeutet, der andere der Reste $R_1$ und $R_2$ für die Gruppe der Formel

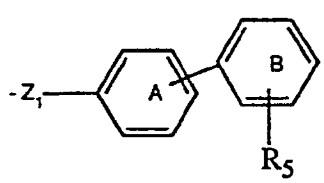

(Ia)

steht, in der $Z_1$ für Alkylen, O, $S(O)_m$ oder N(R) steht, $R_5$ Carboxy, Halogenalkansulfonylamino, $SO_3H$, $PO_2H_2$, $PO_3H_2$ oder 5-Tetrazolyl bedeutet und die Ringe A und B unabhängig voneinander gegebenenfalls substituiert sind durch einen aliphatischen Kohlenwasserstoffrest, der gegebenenfalls durch O unterbrochen und gegebenenfalls durch Hydroxy oder Halogen substituiert ist, gegebenenfalls durch einen aliphatischen Alkohol verethertes Hydroxy, Halogen, gegebenenfalls verestertes oder amidiertes Carboxy oder 5-Tetrazolyl, und entweder $R_3$ Halogen, Acyl, einen aromatischen Kohlenwasserstoffrest, gegebenenfalls verestertes oder amidiertes Carboxy, Cyano, $SO_3H$, $PO_3H_2$, $PO_2H_2$, 5-Tetrazolyl, gegebenenfalls substituiertes Sulfamoyl oder Acylamino bedeutet oder für $-Z_2-R'$ steht, worin $Z_2$ für eine Bindung oder für O, $S(O)_m$ oder N(R) steht und R' Wasserstoff oder einen aliphatischen Kohlenwasserstoffrest bedeutet, der gegebenenfalls durch O oder $S(O)_m$ unterbrochen ist und gegebenenfalls durch Halogen, Hydroxy, gegebenenfalls substituiertes Amino oder gegebenenfalls verestertes oder amidiertes Carboxy substituiert ist, und $R_4$ einen gegebenenfalls durch O oder $S(O)_m$ unterbrochenen aliphatischen Kohlenwasserstoffrest bedeutet, der gegebenenfalls durch gegebenenfalls verestertes oder amidiertes Carboxy, gegebenenfalls mit einem aromatischen Alkohol verethertes Hydroxy, gegebenenfalls substituiertes Amino, $S(O)_m$-R oder einen aromatischen Kohlenwasserstoffrest substituiert ist, oder $R_3$ und $R_4$ gemeinsam für Alkylen stehen,

wobei R jeweils Wasserstoff oder einen aliphatischen Kohlenwasserstoffrest bedeutet und m jeweils für 0, 1 oder 2 steht, in freier Form oder in Salzform, dadurch gekennzeichnet, dass man in einer Verbindung der Formel

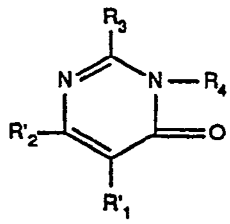

(IIa),

worin einer der Reste $R'_1$ und $R'_2$ für die Gruppe der Formel

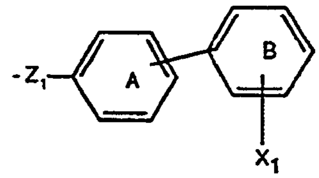

(IIb)

und $X_1$ für einen in $R_5$ überführbaren Rest steht, oder einem Salz davon $X_1$ in $R_5$ überführt und, wenn erwünscht, eine verfahrensgemäss oder auf andere Weise erhältliche Verbindung I in freier Form oder in Salzform in eine andere Verbindung I überführt, ein verfahrensgemäss erhältliches Gemisch von Isomeren auftrennt und das gewünschte Isomere isoliert und/oder eine verfahrensgemäss erhältliche freie Verbindung I in ein Salz oder ein verfahrensgemäss erhältliches Salz einer Verbindung I in die freie Verbindung I oder in ein anderes Salz überführt

25. Verfahren zur Herstellung eines pharmazeutischen Präparats gemäss einem der Ansprüche 20 bis 23, dadurch gekennzeichnet, dass man den Wirkstoff, gegebenenfalls unter Beimischung von üblichen pharmazeutischen Hilfsstoffen, zu einem pharmazeutischen Präparat verarbeitet.

26. Verfahren gemäss Anspruch 25 zur Herstellung eines antihypertensiv wirksamen pharmazeutischen Präparats gemäss Anspruch 22 oder 23, dadurch gekennzeichnet, dass man einen antihypertensiv wirksamen Wirkstoff wählt.

27. Verwendung einer Verbindung gemäss einem der Ansprüche 1 bis 19, in freier Form oder in Form eines pharmazeutisch verwendbaren Salzes, zur Herstellung eines pharmazeutischen Präparats.

28. Verwendung einer Verbindung gemäss einem der Ansprüche 1 bis 19, in freier Form oder in Form eines pharmazeutisch verwendbaren Salzes, zur Herstellung eines pharmazeutischen Präparats auf nicht-chemischem Wege.

29. Verwendung einer Verbindung gemäss Anspruch 27 oder 28 zur Herstellung eines Antihypertensivums.

**Patentansprüche für folgenden Vertragsstaaten : ES und GR**

1. Verfahren zur Herstellung einer Verbindung der Formel

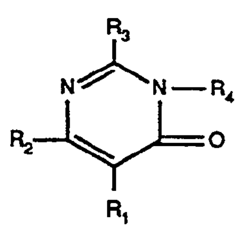

(I),

worin einer der Reste $R_1$ und $R_2$ einen gegebenenfalls durch Halogen oder Hydroxy substituierten aliphatischen Kohlenwasserstoffrest oder einen cycloaliphatischen oder araliphatischen Kohlenwasserstoffrest bedeutet, der andere der Reste $R_1$ und $R_2$ für die Gruppe der Formel

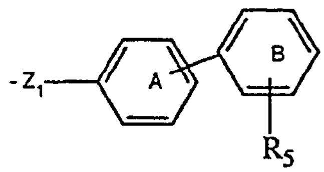

(Ia)

steht, in der $Z_1$ für Alkylen, O, $S(O)_m$ oder N(R) steht, $R_5$ Carboxy, Halogenalkansulfonylamino, $SO_3H$, $PO_2H_2$, $PO_3H_2$ oder 5-Tetrazolyl bedeutet und die Ringe A und B unabhängig voneinander gegebenenfalls substituiert sind durch einen aliphatischen Kohlenwasserstoffrest, der gegebenenfalls durch O unterbrochen und gegebenenfalls durch Hydroxy oder Halogen substituiert ist, gegebenenfalls durch einen aliphatischen Alkohol verethertes Hydroxy, Halogen, gegebenenfalls verestertes oder amidiertes Carboxy oder 5-Tetrazolyl, und entweder $R_3$ Halogen, Acyl, einen aromatischen Kohlenwasserstoffrest, gegebenenfalls verestertes oder amidiertes Carboxy, Cyano, $SO_3H$, $PO_3H_2$, $PO_2H_2$, 5-Tetrazolyl, gegebenenfalls substituiertes Sulfamoyl oder Acylamino bedeutet oder für $-Z_2-R'$ steht, worin $Z_2$ für eine Bindung oder für O, $S(O)_m$ oder N(R) steht und R' Wasserstoff oder einen aliphatischen Kohlenwasserstoffrest bedeutet, der gegebenenfalls durch O oder $S(O)_m$ unterbrochen ist und gegebenenfalls durch Halogen, Hydroxy, gegebenenfalls substituiertes Amino oder gegebenenfalls verestertes oder amidiertes Carboxy substituiert ist, und $R_4$ einen gegebenenfalls durch O oder $S(O)_m$ unterbrochenen aliphatischen Kohlenwasserstoffrest bedeutet, der gegebenenfalls durch gegebenenfalls verestertes oder amidiertes Carboxy, gegebenenfalls mit einem aromatischen Alkohol verethertes Hydroxy, gegebenenfalls substituiertes Amino, $S(O)_m$-R oder einen aromatischen Kohlenwasserstoffrest substituiert ist, oder $R_3$ und $R_4$ gemeinsam für Alkylen stehen, wobei R jeweils Wasserstoff oder einen aliphatischen Kohlenwasserstoffrest bedeutet und m jeweils für 0, 1 oder 2 steht, in freier Form oder in Salzform, dadurch gekennzeichnet, dass man in einer Verbindung der Formel

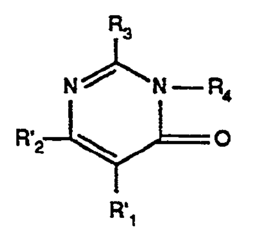

(IIa),

worin einer der Reste $R'_1$ und $R'_2$ für die Gruppe der Formel

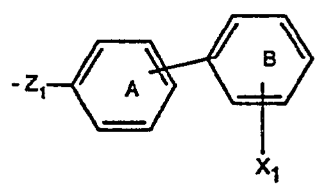

(IIb)

und $X_1$ für einen in $R_5$ überführbaren Rest steht, oder einem Salz davon $X_1$ in $R_5$ überführt und, wenn erwünscht, eine verfahrensgemäss oder auf andere Weise erhältliche Verbindung I in freier Form oder in Salzform in eine andere Verbindung I überführt, ein verfahrensgemäss erhältliches Gemisch von Isomeren auftrennt und das gewünschte Isomere isoliert und/oder eine verfahrensgemäss erhältliche freie Verbindung I in ein Salz oder ein verfahrensgemäss erhältliches Salz einer Verbindung I in die freie Verbindung I oder in ein anderes Salz überführt.

2. Verfahren gemäss Anspruch 1 zur Herstellung einer Verbindung der Formel I, worin einer der Reste $R_1$ und $R_2$ jeweils gegebenenfalls durch Halogen oder Hydroxy substituiertes Niederalkyl, Niederalkenyl oder Niederalkinyl, jeweils 3- bis 7-gliedriges Cycloalkyl oder Cycloalkenyl, Phenylniederalkyl, Phenylniederalkenyl oder Phenylniederalkinyl bedeutet, der andere der Reste $R_1$ und $R_2$ für die Gruppe Ia steht, in der $Z_1$ für Methylen, Niederalkylen, O, $S(O)_m$ oder N(R) steht, $R_5$ Carboxy, Halogenniederalkansulfonylamino, $SO_3H$, $PO_2H_2$, $PO_3H_2$ oder 5-Tetrazolyl bedeutet und die Ringe A und B unabhängig voneinander gegebenenfalls substituiert sind durch Halogen, durch jeweils gegebenenfalls durch Halogen oder Hydroxy substituiertes Niederalkyl, Niederalkenyl, Niederalkinyl, Niederalkoxyniederalkyl, Niederalkoxyniederalkenyl, Niederalkoxyniederalkinyl, Niederalkenyloxyniederalkyl, Niederalkenyloxyniederalkenyl, Niederalkenyloxyniederalkinyl oder Niederalkoxyniederalkoxyniederalkyl, durch Hydroxy, durch Niederalkoxy, durch Niederalkenyloxy, durch Carboxy, welches gegebenenfalls durch einen Alkohol verestert ist, der sich von Niederalkyl, Niederalkenyl, Niederalkinyl, Niederalkoxyniederalkyl, Niederalkoxyniederalkenyl oder Niederalkoxyniederalkinyl ableitet, durch Carbamoyl, in welchem die Aminogruppe gegebenenfalls durch Niederalkyl, Niederalkenyl, Niederalkinyl, Phenylniederalkyl, Phenylniederalkenyl oder Phenylniederalkinyl unabhängig voneinander mono- oder di-substituiert oder durch Niederalkylen oder Niederalkylenoxyniederalkylen disubstituiert ist, oder durch 5-Tetrazolyl, und entweder $R_3$ Halogen, Niederalkanoyl, gegebenenfalls substituiertes Phenyl, Carboxy, welches gegebenenfalls durch einen Alkohol verestert ist, der sich von Niederalkyl, Niederalkenyl, Niederalkinyl, Niederalkoxyniederalkyl, Niederalkoxyniederalkenyl oder Niederalkoxyniederalkinyl ableitet, Carbamoyl, in welchem die Aminogruppe gegebenenfalls durch Niederalkyl, Niederalkenyl, Niederalkinyl, Phenylniederalkyl, Phenylniederalkenyl oder Phenylniederalkinyl unabhängig voneinander mono- oder di-substituiert ist oder durch Niederalkylen oder Niederalkylenoxyniederalkylen disubstituiert ist, Cyano, $SO_3H$, $PO_2H_2$, $PO_3H_2$, 5-Tetrazolyl, Sulfamoyl, in dem die Aminogruppe gegebenenfalls durch Niederalkyl mono- oder disubstituiert ist, Niederalkansulfonylamino, Halogenniederalkansulfonylamino, Niederalkanoylamino, gegebenenfalls substituiertes Benzoylamino oder gegebenenfalls substituiertes Benzolsulfonylamino bedeutet oder für $-Z_2-R'$ steht, worin $Z_2$ für eine Bindung oder für O, S $(O)_m$ oder N(R) steht und R' Wasserstoff oder jeweils gegebenenfalls durch Hydroxy, Halogen, Amino, Niederalkylenamino, Niederalkylenoxyniederalkylenamino, Niederalkylamino, Niederalkenylamino, Niederalkinylamino, Phenylniederalkylamino, Phenylniederalkenylamino, Phenylniederalkinylamino, Diniederalkylamino, N-Niederalkyl-N-phenylniederalkyl-amino, Di(phenylniederalkyl)amino, Carboxy, welches gegebenenfalls durch einen Alkohol verestert ist, der sich von Niederalkyl, Niederalkenyl, Niederalkinyl, Niederalkoxyniederalkyl, Niederalkoxyniederalkenyl oder Niederalkoxyniederalkinyl ableitet, oder Carbamoyl, in welchem die Aminogruppe gegebenenfalls durch Niederalkyl, Niederalkenyl, Niederalkinyl, Phenylniederalkyl, Phenylniederalkenyl oder Phenylniederalkinyl unabhängig voneinander mono- oder di-substituiert oder durch Niederalkylen oder Niederalkylenoxyniederalkylen disubstituiert ist, substituiertes Niederalkyl, Niederalkenyl, Niederalkinyl, Niederalkoxyniederalkyl, Niederalkoxyniederalkenyl, Niederalkoxyniederalkinyl, Niederalkenyloxyniederalkyl, Niederalkenyloxyniederalkenyl, Niederalkenyloxyniederalkinyl, Niederalkoxyniederalkoxyniederalkyl, Niederalkylthio-niederalkyl, -niederalkenyl oder -niederalkinyl, Niederalkan-sulfinylniederalkyl oder -sulfonylniederalkyl, Niederalkenyl-thioniederalkyl, -sulfinylniederalkyl oder -sulfonylniederalkyl oder Niederalkinyl-thioniederalkyl, -sulfinylniederalkyl oder -sulfonylniederalkyl bedeutet, und $R_4$ jeweils gegebenenfalls durch Hydroxy, $S(O)_m$-R, jeweils gegebenenfalls substituiertes Phenyl, Phenoxy oder Naphthyloxy, Amino, Niederalkylenamino, Niederalkylenoxyniederalkylenamino, Niederalkylamino, Niederalkenylamino, Niederalkinylamino, Phenylniederalkylamino, Phenylniederalkenylamino, Phenylniederalkinylamino, Diniederalkylamino, N-Niederalkyl-N-phenylniederalkyl-amino, Di(phenylniederalkyl)amino, Carboxy, welches gegebenenfalls durch einen Alkohol verestert ist, der sich von Niederalkyl, Niederalkenyl, Niederalkinyl, Niederalkoxyniederalkyl, Niederalkoxyniederalkenyl oder Niederalkoxyniederalkinyl ableitet, oder Carbamoyl, in welchem die Aminogruppe gegebenenfalls durch Niederalkyl, Niederalkenyl, Niederalkinyl, Phenylniederalkyl, Phenylniederalkenyl oder Phenylniederalkinyl unabhängig voneinander mono- oder di-substituiert oder durch Niederalkylen oder Niederalkylenoxyniederalkylen disubstituiert ist, substituiertes Niederalkyl, Niederalkenyl, Niederalkinyl, Niederalkoxyniederalkyl, Niederalkoxyniederalkenyl, Niederalkoxyniederalkinyl, Niederalkenyloxyniederalkyl, Niederalkenyloxyniederalkenyl, Niederalkenyloxyniederalkinyl, Niederalkoxyniederalkoxyniederalkyl, Niederalkylthio-niederalkyl, -niederalkenyl oder -niederalkinyl, Niederalkan-sulfinylniederalkyl oder -sulfonylniederalkyl, Niederalkenyl-thioniederalkyl, -sulfinylniederalkyl oder -sulfonylniederalkyl oder Niederalkinyl-thioniederalkyl, -sulfinylniederalkyl oder -sulfonylniederalkyl bedeutet, oder $R_3$ und $R_4$ gemeinsam für Methylen oder Niederalkylen stehen, wobei R jeweils Wasserstoff, Niederalkyl, Niederalkenyl oder Niederalkinyl bedeutet, m jeweils für 0, 1 oder 2 steht und aromatische Reste jeweils gegebenenfalls durch Niederalkyl, Niederalkoxy, Halogen, Trifluormethyl und/oder Hydroxy substituiert sind, in freier Form oder in Salzform.

3. Verfahren gemäss Anspruch 1 zur Herstellung einer Verbindung der Formel I, worin $R_1$ für die Gruppe Ia steht, in der $Z_1$ für Methylen, Niederalkylen, O, $S(O)_m$ oder N(R) steht, $R_5$ Carboxy oder 5-Tetrazolyl bedeutet und die Ringe A und B unabhängig voneinander gegebenenfalls durch Halogen, Niederalkyl, Niederalkoxy, Carboxy, Niederalkoxycarbonyl oder 5-Tetrazolyl substituiert sind, $R_2$ jeweils gegebenenfalls durch Hydroxy oder Halogen substituiertes Niederalkyl oder Niederalkenyl bedeutet und entweder $R_3$ Halogen, Carboxy, welches gegebenenfalls durch einen Alkohol verestert ist, der sich von Niederalkyl oder Niederalkoxyniederalkyl ableitet, Carbamoyl, Cyano, $PO_3H_2$, 5-Tetrazolyl, Niederalkansulfamoyl, Niederalkanoylamino oder Niederalkansulfonylamino bedeutet oder für $-Z_2-R'$ steht, worin $Z_2$ für eine Bindung oder für O, S $(O)_m$ oder N(R) steht und R' Wasserstoff oder jeweils gegebenenfalls durch Carboxy, Niederalkoxycarbonyl, Niederalkoxyniederalkoxycarbonyl oder Hydroxy substituiertes Niederalkyl oder Niederalkoxyniederalkyl bedeutet, und $R_4$ Niederalkyl, Niederalkenyl, Niederalkoxyniederalkyl, Carboxyniederalkyl, Niederalkoxycarbonylniederalkyl, Niederalkoxyniederalkoxycarbonylniederalkyl, Carbamoyl-, Niederalkylcarbamoyl- oder Diniederalkylcarbamoyl-niederalkyl, Hydroxyniederalkyl, gegebenenfalls substituiertes Phenoxyniederalkyl, Amino-, Niederalkylamino- oder Diniederalkylamino-niederalkyl oder gegebenenfalls substituiertes Phenylniederalkyl bedeutet, oder $R_3$ und $R_4$ gemeinsam für Methylen oder Niederalkylen stehen, wobei R jeweils Wasserstoff oder Niederalkyl bedeutet, m jeweils 0, 1 oder 2 bedeutet und aromatische Reste jeweils gegebenenfalls durch Niederalkyl, Niederalkoxy, Halogen, Trifluormethyl und/oder Hydroxy substituiert sind, in freier Form oder in Salzform.

4. Verfahren gemäss Anspruch 1 zur Herstellung einer Verbindung der Formel I, worin $R_1$ für die Gruppe Ia steht, in der $Z_1$ für Methylen, Niederalkylen, O oder $S(O)_m$ steht, $R_5$ Carboxy oder 5-Tetrazolyl bedeutet und die Ringe A und B unabhängig voneinander gegebenenfalls durch Niederalkyl, Halogen oder Niederalkoxy substituiert sind, $R_2$ jeweils gegebenenfalls durch Hydroxy oder Halogen substituiertes Niederalkyl oder Niederalkenyl bedeutet und entweder $R_3$ Halogen, Carboxy, Niederalkoxycarbonyl, $PO_3H_2$, 5-Tetrazolyl, Niederalkanoylamino, Niederalkansulfonylamino, Wasserstoff, Niederalkyl, Niederalkoxyniederalkyl, Carboxyniederalkyl, Niederalkoxycarbonylniederalkyl, Hydroxyniederalkyl, Hydroxyniederalkoxyniederalkyl, Hydroxy, Niederalkoxy, Niederalkoxyniederalkoxy, Carboxyniederalkoxy, Niederalkoxycarbonylniederalkoxy, Hydroxyniederalkoxy, Mercapto, Niederalkylthio, Niederalkansulfinyl, Niederalkansulfonyl, Amino, Niederalkylamino oder Diniederalkylamino bedeutet und $R_4$ Niederalkyl, Hydroxyniederalkyl, Carboxyniederalkyl oder Niederalkoxycarbonylniederalkyl bedeutet oder $R_3$ und $R_4$ gemeinsam für Methylen oder Niederalkylen stehen, wobei m für 0, 1 oder 2 steht und mit "nieder" bezeichnete Teilstrukturen jeweils bis und mit 7 C-Atome umfassen, in freier Form oder in Salzform.

5. Verfahren gemäss Anspruch 1 zur Herstellung einer Verbindung der Formel I, worin $R_1$ für die Gruppe Ia steht, in der $Z_1$ für Methylen oder Niederalkylen steht, $R_5$ Carboxy oder 5-Tetrazolyl bedeutet und die Ringe A und B unabhängig voneinander gegebenenfalls durch Niederalkyl, Halogen, mit einer Atomnummer bis und mit 35 oder Niederalkoxy substituiert sind, $R_2$ Niederalkyl bedeutet, $R_3$ Halogen, mit einer Atomnummer bis und mit 35, Wasserstoff, Niederalkyl, Niederalkoxyniederalkyl, Carboxyniederalkyl, Niederalkoxycarbonylniederalkyl, Hydroxyniederalkyl, Hydroxyniederalkoxyniederalkyl, Hydroxy, Niederalkoxy, Niederalkoxyniederalkoxy, Carboxyniederalkoxy, Niederalkoxycarbonylniederalkoxy, Hydroxyniederalkoxy oder Niederalkoxyniederalkoxy bedeutet und $R_4$ Niederalkyl, Hydroxyniederalkyl, Carboxyniederalkyl oder Niederalkoxycarbonylniederalkyl bedeutet, wobei mit "nieder" bezeichnete Teilstrukturen jeweils bis und mit 4 C-Atome umfassen, in freier Form oder in Salzform.

6. Verfahren gemäss einem der Ansprüche 1 bis 5 zur Herstellung einer Verbindung der Formel I, worin $R_1$ für die Gruppe der Formel

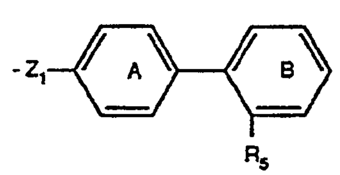

(Ib)

steht, in freier Form oder in Salzform.

7. Verfahren gemäss Anspruch 1 zur Herstellung einer Verbindung der Formel I, worin $R_1$ für die Gruppe Ib steht, in der $Z_1$ Methylen bedeutet, $R_5$ Carboxy oder 5-Tetrazolyl bedeutet und die Ringe A und B unsubstituiert sind, $R_2$ $C_3$-$C_7$-Alkyl bedeutet, $R_3$ Wasserstoff oder $C_1$-$C_4$-Alkyl bedeutet und $R_4$ $C_1$-$C_4$-Alkyl, Carboxy-$C_1$-$C_4$-alkyl, $C_1$-$C_4$-Alkoxycarbonyl-$C_1$-$C_4$-alkyl oder Hydroxy-$C_1$-$C_4$-alkyl bedeutet, in freier Form oder

in Salzform.

8. Verfahren gemäss Anspruch 1 zur Herstellung einer Verbindung der Formel I, worin $R_1$ für die Gruppe Ib steht, in der $Z_1$ Methylen bedeutet, $R_5$ Carboxy oder Tetrazolyl bedeutet und die Ringe A und B unsubstituiert sind, $R_2$ $C_3$-$C_5$-Alkyl bedeutet, $R_3$ Wasserstoff oder $C_1$-$C_4$-Alkyl bedeutet und $R_4$ $C_1$-$C_4$-Alkyl bedeutet, in freier Form oder in Salzform.

9. Verfahren gemäss Anspruch 1 zur Herstellung von 4-(n-Butyl)-1,2-dimethyl-6-oxo-5-[2'-(1H-tetrazol-5-yl)biphenyl-4-ylmethyl]-1,6-dihydro-pyrimidin, in freier Form oder in Salzform.

10. Verfahren gemäss Anspruch 1 zur Herstellung von 4-(n-Butyl)-1-ethyl-2-methyl-6-oxo-5-[2'-(1H-tetrazol-5-yl)biphenyl-4-ylmethyl]-1,6-dihydro-pyrimidin, in freier Form oder in Salzform.

11. Verfahren gemäss Anspruch 1 zur Herstellung von 4-(n-Butyl)-2-methyl-6-oxo-1-(n-propyl)-5-[2'-(1H-tetrazol-5-yl)biphenyl-4-ylmethyl]-1,6-dihydro-pyrimidin, in freier Form oder in Salzform.

12. Verfahren gemäss Anspruch 1 zur Herstellung von 4-(n-Butyl)-1-ethoxycarbonyl-methyl-2-methyl-6-oxo-5-[2'-(1H-tetrazol-5-yl)biphenyl-4-ylmethyl]-1,6-dihydro-pyrimidin, in freier Form oder in Salzform.

13. Verfahren gemäss Anspruch 1 zur Herstellung von 4-(n-Butyl)-1-(2-ethoxyethyl)-2-methyl-6-oxo-5-[2'-(1H-tetrazol-5-yl)biphenyl-4-ylmethyl]-1,6-dihydro-pyrimidin,
1,4-Di-(n-butyl)-2-methyl-6-oxo-5-[2'-(1H-tetrazol-5-yl)biphenyl-4-ylmethyl]-1,6-dihydro-pyrimidin,
1-Benzyl-4-(n-butyl)-2-methyl-6-oxo-5-[2'-(1H-tetrazol-5-yl)biphenyl-4-ylmethyl]-1,6-dihydro-pyrimidin,
4-(n-Butyl)-2-methyl-6-oxo-1-(2-phenylethyl)-5-[2'-(1H-tetrazol-5-yl)biphenyl-4-ylmethyl]-1,6-dihydro-pyrimidin,
4-(n-Butyl)-1-(2-hydroxyethyl)-2-methyl-6-oxo-5-[2'-(1H-tetrazol-5-yl)biphenyl-4-ylmethyl]-1,6-dihydro-pyrimidin oder
4-(n-Butyl)-1-methyl-6-oxo-2-(n-propyl)-5-[2'-(1H-tetrazol-5-yl)biphenyl-4-ylmethyl]-1,6-dihydro-pyrimidin,
jeweils in freier Form oder in Salzform.

14. Verfahren gemäss Anspruch 1 zur Herstellung von 2,4-Di-(n-butyl)-1-methyl-6-oxo-5-[2'-(1H-tetrazol-5-yl)biphenyl-4-ylmethyl]-1,6-dihydro-pyrimidin,
4-(n-Butyl)-2-isopropyl-1-methyl-6-oxo-5-[2'-(1H-tetrazol-5-yl)biphenyl-4-ylmethyl]-1,6-dihydro-pyrimidin,
4-(n-Butyl)-1,2-diethyl-6-oxo-5-[2'-(1H-tetrazol-5-yl)biphenyl-4-ylmethyl]-1,6-dihydro-pyrimidin,
4-(n-Butyl)-1-ethyl-6-oxo-2-(n-propyl)-5-[2'-(1H-tetrazol-5-yl)biphenyl-4-ylmethyl]-1,6-dihydro-pyrimidin,
2,4-Di-(n-butyl)-1-ethyl-6-oxo-5-[2'-(1H-tetrazol-5-yl)biphenyl-4-ylmethyl]-1,6-dihydro-pyrimidin,
4-(n-Butyl)-1-ethyl-2-isopropyl-6-oxo-5-[2'-(1H-tetrazol-5-yl)biphenyl-4-ylmethyl]-1,6-dihydro-pyrimidin,
4-(n-Butyl)-2-ethyl-6-oxo-1-(n-propyl)-5-[2'-(1H-tetrazol-5-yl)biphenyl-4-ylmethyl]-1,6-dihydro-pyrimidin,
4-(n-Butyl)-1,2-di-(n-propyl)-6-oxo-5-[2'-(1H-tetrazol-5-yl)biphenyl-4-ylmethyl]-1,6-dihydro-pyrimidin,
2,4-Di-(n-butyl)-6-oxo-1-(n-propyl)-5-[2'-(1H-tetrazol-5-yl)biphenyl-4-ylmethyl]-1,6-dihydro-pyrimidin,
4-(n-Butyl)-2-isopropyl-6-oxo-1-(n-propyl)-5-[2'-(1H-tetrazol-5-yl)biphenyl-4-ylmethyl]-1,6-dihydro-pyrimidin,
1,4-Di-(n-butyl)-2-ethyl-6-oxo-5-[2'-(1H-tetrazol-5-yl)biphenyl-4-ylmethyl]-1,6-dihydro-pyrimidin,
1,4-Di-(n-butyl)-6-oxo-2-(n-propyl)-5-[2'-(1H-tetrazol-5-yl)biphenyl-4-ylmethyl]-1,6-dihydro-pyrimidin,
6-Oxo-5-[2'-(1H-tetrazol-5-yl)biphenyl-4-ylmethyl]-1,2,4-tri-(n-butyl)-1,6-dihydro-pyrimidin,
1,4-Di-(n-butyl)-2-isopropyl-6-oxo-5-[2'-(1H-tetrazol-5-yl)biphenyl-4-ylmethyl]-1,6-dihydro-pyrimidin,
1,2-Dimethyl-6-oxo-4-(n-propyl)-5-(2'-carboxybiphenyl-4-ylmethyl)-1,6-dihydro-pyrimidin,
4-(n-Butyl)-1-carboxymethyl-2-methyl-6-oxo-5-[2'-(1H-tetrazol-5-yl)biphenyl-4-ylmethyl]-1,6-dihydro-pyrimidin,
4-(n-Butyl)-5-(2'-carboxybiphenyl-4-ylmethyl)-1,2-dimethyl-6-oxo-1,6-dihydro-pyrimidin oder
4-(n-Butyl)-2-ethyl-1-methyl-6-oxo-5-[2'-(1H-tetrazol-5-yl)biphenyl-4-ylmethyl]-1,6-dihydro-pyrimidin,
jeweils in freier Form oder in Salzform.

15. Verfahren gemäss Anspruch 1 zur Herstellung von 4-(n-Butyl)-2-methyl-1-[N,N-(3-oxapent-1,5-ylen)aminocarbonylmethyl]-6-oxo-5-[2'-(1H-tetrazol-5-yl)biphenyl-4-ylmethyl]-1,6-dihydro-pyrimidin,
4-(n-Butyl)-1-[2-(2-methoxyethoxy)ethyl]-2-methyl-6-oxo-5-[2'-(1H-tetrazol-5-yl)biphenyl-4-ylmethyl]-1,6-dihydro-pyrimidin,
1-Benzylaminocarbonylmethyl-4-(n-butyl)-2-methyl-6-oxo-5-[2'-(1H-tetrazol-5-yl)biphenyl-4-ylmethyl]-1,6-dihydro-pyrimidin,
4-(n-Butyl)-2-methyl-6-oxo-1-(2-phenylethylaminocarbonylmethyl)-5-[2'-(1H-tetrazol-5-yl)biphenyl-4-ylm ethyl]-1,6-dihydro-pyrimidin,
4-(n-Butyl)-1-(n-butylaminocarbonylmethyl)-2-methyl-6-oxo-5-[2'-(1H-tetrazol-5-yl)biphenyl-4-ylmethyl]-1 ,6-dihydro-pyrimidin,
4-(n-Butyl)-2-methyl-1-methylaminocarbonylmethyl-6-oxo-5-[2'-(1H-tetrazol-5-yl)biphenyl-4-ylmethyl]-1, 6-dihydro-pyrimidin,
4-(n-Butyl)-1-dimethylaminocarbonylmethyl-2-methyl-6-oxo-5-[2'-(1H-tetrazol-5-yl)biphenyl-4-ylmethyl]-1,6-dihydro-pyrimidin,
4-(n-Butyl)-6-oxo-1,2-tetramethylen-5-[2'-(1H-tetrazol-5-yl)biphenyl-4-ylmethyl]-1,6-dihydro-pyrimidin oder
4-(n-Butyl)-6-oxo-5-[2'-(1H-tetrazol-5-yl)biphenyl-4-ylmethyl]-1,2-trimethylen-1,6-dihydro-pyrimidin,
jeweils in freier Form oder in Salzform.

16. Verfahren zur Herstellung eines pharmazeutischen Präparats, dadurch gekennzeichnet, dass man in einer Verbindung der Formel

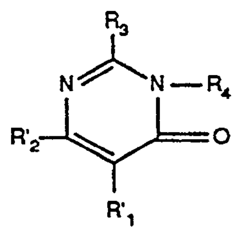

(IIa),

worin einer der Reste $R'_1$ und $R'_2$ für die Gruppe der Formel

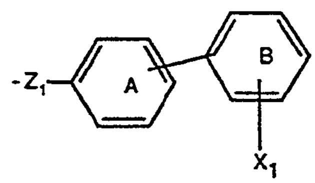

(IIb)

und $X_1$ für einen in $R_5$ überführbaren Rest steht, oder einem Salz davon $X_1$ in $R_5$ überführt und, wenn erwünscht, eine verfahrensgemäss oder auf andere Weise erhältliche Verbindung I in freier Form oder in pharmazeutisch verwendbares Salzform in eine andere Verbindung I überführt, ein verfahrensgemäss erhältliches Gemisch von Isomeren auftrennt und das gewünschte Isomere isoliert und/oder eine verfahrensgemäss erhältliche freie Verbindung I in ein pharmazeutisch verwendbares Salz oder ein verfahrensgemäss erhältliches pharmazeutisch verwendbares Salz einer Verbindung I in die freie Verbindung I oder in ein anderes pharmazeurisch verwendbares Salz überführt und eine auf diese Weise erhaltene Verbindung der Formel

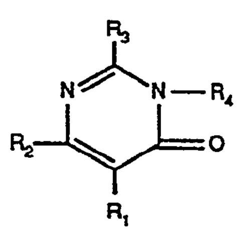

(I),

worin einer der Reste $R_1$ und $R_2$ einen gegebenenfalls durch Halogen oder Hydroxy substituierten aliphatischen Kohlenwasserstoffrest oder einen cycloaliphatischen oder araliphatischen Kohlenwasserstoffrest bedeutet, der andere der Reste $R_1$ und $R_2$ für die Gruppe der Formel

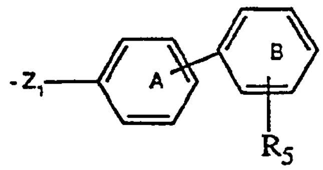

(Ia)

steht, in der $Z_1$ für Alkylen, O, $S(O)_m$ oder N(R) steht, $R_5$ Carboxy, Halogenalkansulfonylamino, $SO_3H$, $PO_2H_2$, $PO_3H_2$ oder 5-Tetrazolyl bedeutet und die Ringe A und B unabhängig voneinander gegebenenfalls substituiert sind durch einen aliphatischen Kohlenwasserstoffrest, der gegebenenfalls durch O unterbrochen und gegebenenfalls durch Hydroxy oder Halogen substituiert ist, gegebenenfalls durch einen aliphatischen Alkohol verethertes Hydroxy, Halogen, gegebenenfalls verestertes oder amidiertes Carboxy oder 5-Tetrazolyl, und entweder $R_3$ Halogen, Acyl, einen aromatischen Kohlenwasserstoffrest, gegebenenfalls verestertes oder amidiertes Carboxy, Cyano, $SO_3H$, $PO_3H_2$, $PO_2H_2$, 5-Tetrazolyl, gegebenenfalls substituiertes Sulfamoyl oder Acylamino bedeutet oder für $-Z_2-R'$ steht, worin $Z_2$ für eine Bindung oder für O, $S(O)_m$ oder N(R) steht und R' Wasserstoff oder einen aliphatischen Kohlenwasserstoffrest bedeutet, der gegebenenfalls durch O oder $S(O)_m$ unterbrochen ist und gegebenenfalls durch Halogen, Hydroxy, gegebenenfalls substituiertes Amino oder gegebenenfalls verestertes oder amidiertes Carboxy substituiert ist, und $R_4$ einen gegebenenfalls durch O oder $S(O)_m$ unterbrochenen aliphatischen Kohlenwasserstoffrest bedeutet, der gegebenenfalls durch gegebenenfalls verestertes oder amidiertes Carboxy, gegebenenfalls mit einem aromatischen Alkohol verethertes Hydroxy, gegebenenfalls substituiertes Amino, $S(O)_m$-R oder einen aromatischen Kohlenwasserstoffrest substituiert ist, oder $R_3$ und $R_4$ gemeinsam für Alkylen stehen, wobei R jeweils Wasserstoff oder einen aliphatischen Kohlenwasserstoffrest bedeutet und m jeweils für 0, 1 oder 2 steht, in freier Form oder in pharmazeutisch verwendbarer Salzform, gegebenenfalls unter Beimischung von üblichen pharmazeutischen Hilfsstoffen, zu einem pharmazeutischen Präparat verarbeitet.

17. Verfahren zur Herstellung eines pharmazeutischen Präparats, dadurch gekennzeichnet, dass man eine Verbindung, erhältlich gemäss einem der Anspruche 1 bis 15, in freier Form oder in Form eines pharmazeutisch verwendbaren Salzes, gegebenenfalls unter Beimischung von üblichen pharmazeutischen Hilfsstoffen, zu einem pharmazeutischen Präparat verarbeitet.

18. Verfahren zur Herstellung eines pharmazeutischen Präparats gemäss Anspruch 17, dadurch gekennzeichnet, dass man eine Verbindung, erhältich gemäss einem der Ansprüche 5 und 9 bis 14, in freier Form oder in Form eines pharmazeutisch verwendbaren Salzes, gegebenenfalls unter Beimischung von üblichen pharmazeutischen Hilfsstoffen, zu einem pharmazeutischen Präparat verarbeitet.

19. Verfahren gemäss einem der Ansprüche 16 bis 18 zur Herstellung eines antihypertensiv wirksamen pharmazeutischen Präparats, dadurch gekennzeichnet, dass man einen antihypertensiv wirksamen Wirkstoff wählt.

20. Verfahren gemäss Anspruch 18 zur Herstellung eines antihypertensiv wirksamen pharmazeutischen Präparats, dadurch gekennzeichnet, dass man einen antihypertensiv wirksamen Wirkstoff wählt.

21. Verwendung einer Verbindung, erhältlich gemäss einem der Ansprüche 1 bis 15, in freier Form oder in Form eines pharmazeutisch verwendbaren Salzes, zur Herstellung eines pharmazeutischen Präparats.

22. Verwendung einer Verbindung, erhältlich gemäss einem der Anspruche 1 bis 15, in freier Form oder in Form eines pharmazeutisch verwendbaren Salzes, zur Herstellung eines pharmazeutischen Präparats auf nichtchemischem Wege.

23. Verwendung einer Verbindung gemäss Anspruch 21 oder 22 zur Herstellung eines Antihypertensivums.